# EUROPEAN PATENT SPECIFICATION

(11) **EP 2 454 596 B1**
(45) Date of publication and mention of the grant of the patent: **11.06.2014**
(21) Application number: 10732313.1
(22) Date of filing: 14.07.2010
(51) Int. Cl.: G01N 33/574

(54) **FLAP ENDONUCLEASE-1 AS A MARKER FOR CANCER**
FLAP-ENDONUKLEASE-1 ALS MARKER FÜR KREBS
Flap endonucléase-1 en tant que marqueur pour le cancer

(30) Priority: 16.07.2009 EP 09165636
(43) Date of publication of application: 23.05.2012
(73) Proprietor: Roche Diagnostics GmbH, 68305 Mannheim (DE); F.Hoffmann-La Roche AG, 4070 Basel (CH)
(72) Inventor: WILD, Norbert, 82538 Geretsried/Gelting (DE); HAGMANN, Marie Luise, 82377 Penzberg (DE); KARL, Johann, 82380 Peissenberg (DE); RIEDLINGER, Julia, 85521 Ottobrunn (DE); ROESSLER, Markus, 82110 Germering (DE); TACKE, Michael, 80689 München (DE)
(74) Representative: Burger, Alexander
(86) International application number: PCT/EP2010/004277
(87) International publication number: WO 2011/006642

(56) References cited:
- US-A1- 2007 077 556
- SATO MITSUO ET AL: "Increased expression and no mutation of the Flap endonuclease (FEN1) gene in human lung cancer." ONCOGENE, vol. 22, no. 46, 16 October 2003 (2003-10-16), pages 7243-7246, XP002543517 ISSN: 0950-9232

## Description

The present invention relates to a method and use as defined in claims 1-11.

Cancer remains a major public health challenge despite progress in detection and therapy. Cancer cells are characterized by the production of cancer-associated marker proteins. Cancer-associated proteins are found both in the tissues and in the bodily fluids of an individual who carries cancer cells. Their levels usually are low at the early stages of the carcinogenic progress and increase during the disease's progression and only in rare cases proteins are observed showing a decreased level in the course of disease progression. The sensitive detection of these proteins is an advantageous and promising approach for the diagnosis of cancer, in particular in an early stage diagnosis of cancer. The most prevalent cancer types are breast cancer (BC), lung cancer (LC) and colorectal cancer (CRC).

The most important therapeutic approaches for solid tumors are:
a) surgical resection of the tumor,
b) chemotherapy,
c) radiation therapy,
d) treatment with biologicals, like anti-tumor antibodies or anti-angiogenic antibodies and
e) a combination of the above methods.

Surgical resection of the tumors is widely accepted as a first line treatment for early stage solid tumors. Most cancers, however, are detected only when they become symptomatic, i.e. when patients already are in a rather late stage of disease progression.

The staging of cancer is the classification of the disease in terms of extent, progression, and severity. It groups cancer patients so that generalizations can be made about prognosis and the choice of therapy.

The different stages of CRC used to be classified according to Dukes' stages A to D. Today, the TNM system is the most widely used classification of the anatomical extent of cancer. It represents an internationally accepted, uniform staging system. There are three basic variables: T (the extent of the primary tumor), N (the status of regional lymph nodes) and M (the presence or absence of distant metastases). The TNM criteria are published by the UICC (International Union Against Cancer), Sobin, L.H., Wittekind, Ch. (eds.), TNM Classification of Malignant Tumours, sixth edition (2002)). Once the TNM status is determined the patients are grouped into disease stages that are denoted by Roman numerals ranging form I to IV with IV being the most advanced disease stage. TNM staging and UICC disease stages correspond to each other as shown in the following table taken from Sobin and Wittekind (eds.), supra.

**Table 1: Interrelation of TNM staging and UICC disease stages**

| UICC disease stage | T staging | N staging | M staging |
|---|---|---|---|
| Stage 0 | Tis | N0 | M0 |
| Stage I | T1, T2 | N0 | M0 |
| Stage IIA | T3 | N0 | M0 |
| Stage IIB | T4 | N0 | M0 |
| Stage IIIA | T1, T2 | N1 | M0 |
| Stage IIIB | T3, T4 | N1 | M0 |
| Stage IIIC | Any T | N2 | M0 |
| Stage IV | Any T | Any N | M1 |

What is especially important is that early diagnosis cancer, e.g. of CRC translates to a much better prognosis. In CRC malignant tumors of the colorectum arise from benign tumors, i.e. from adenoma. Therefore, best prognosis have those patients diagnosed at the adenoma stage. Patients diagnosed as early as in stage Tᵢₛ, N0, M0 or T1-3; N0; M0, if treated properly have a more than 90% chance of survival 5 years after diagnosis as compared to a 5-years survival rate of only 10% for patients diagnosed when distant metastases are already present.

Current detection methods including imaging methods, such as X-ray or nuclear resonance imaging in theory might at least partially be appropriate for use as a general screening tool. However, they are very costly and not affordable to health care systems for a general and broad use in mass screenings of large numbers of subjects, particularly for subjects without any tumor symptoms.

Thus, it is an object of the present invention to provide a simple and cost-efficient procedure of tumor assessments, e.g. to identify individuals suspect of having cancer. For this purpose, a general tumor marker which is detectable in body fluids, e.g. blood or serum or plasma or a panel of such markers, would be desirable.

A number of serum tumor markers are already in clinical use. For example the soluble 30 kDa fragment of cytoceratin 19 (CYFRA 21-1), carcinoembryogenic antigen (CEA), neuron-specific enolase (NSE), and squamous cell carcinoma antigen (SCC) are the most prominent LC markers. However, none of them meets the criteria for sensitivity and specificity required for a screening tool (Thomas, L., Labor und Diagnose, TH Books Verlagsgesellschaft, Frankfurt/Main, Germany (2000)).

In order to be of clinical utility, a new diagnostic marker as a single marker should be comparable to other markers known in the art, or better. Or, a new marker should lead to a progress in diagnostic sensitivity and/or specificity either if used alone or in combination with one or more other markers, respectively. The diagnostic sensitivity and/or specificity of a test is best assessed by its receiver-operating characteristics, which will be described in detail below.

Whole blood, serum or plasma are the most widely used sources of sample in clinical routine. The identification of an early tumor marker that would aid in the reliable cancer detection or provide early prognostic information could lead to a method that would greatly aid in the diagnosis and in the management of this disease. Therefore, an urgent clinical need exists to improve the in vitro assessment of cancer. It is especially important to improve the early diagnosis of cancer, since for patients diagnosed early on chances of survival are much higher as compared to those diagnosed at a progressed stage of disease.

The clinical utility of biochemical markers in lung cancer has recently been reviewed (Duffy, M.J., Crit. Rev. Clin. Lab. Sci. 38 (2001) 225-262).

With respect to marker profiles and aiming at improved diagnosis of lung cancer, a method was published (Schneider, J. et al., Int. J. Clin. Oncol. 7 (2002) 145-151) using fuzzy logic based classification algorithms to combine serum levels of CYFRA 21-1, NSE and C-reactive protein (CRP) which is a general inflammation marker. The authors report a sensitivity of 92% at a specificity of 95%. However in this study, for example the sensitivity of CYFRA 21-1 as a single tumor marker is reported to be at 72% at a specificity of 95%, which is significantly higher than in many other reported studies. Duffy, M.J., in Crit. Rev. Clin. Lab. Sci. 38 (2001) 225-262, report a sensitivity of between 46% and 61%. This unusual high performance achieved by Schneider et al., raises some doubts and might be due to several facts. Firstly, the collective of control patients seems to be younger than the patients collective, i.e. the groups are not well age-matched, and the patient collective comprises many late stages. Secondly and even more critical, the performance of the algorithm is checked on the samples of the training set which were used for the determination of the fuzzy logic qualifiers. Hence, these qualifiers are strictly speaking "tailor-made" for this set and not applied to an independent validation set. Under normal circumstances, is has to be expected that the same algorithm applied to a larger, independent, and well balanced validation set will lead to a significantly reduced overall performance.

It was the object of the present invention to investigate whether a biochemical marker can be identified which may be used in assessing cancer disease. In particular, the inventors of the present invention investigated whether a general biochemical marker could be identified for the assessment of cancer in body fluids. In the present invention especially the identification of a biochemical marker for the assessment of endometrial cancer, malignant melanoma, cervix cancer, head and neck cancer, ovarian cancer, colon cancer, bladder cancer, pancreatic cancer, breast cancer, small cell lung cancer, prostate cancer, kidney cancer or non small cell lung cancer was investigated.

Surprisingly, it has been found that use of Flap endonuclease-1 protein (= FEN1) can at least partially overcome some of the problems of the markers presently known in the state of the art.

Surprisingly, it has been found that a increased concentration of FEN1 in the test sample is associated with the occurrence of cancer. It could be shown that FEN1 is a marker which is not specific for a single type of cancer, but a marker for different types of cancer, i.e. a general tumor marker. Since FEN1 appears to be rather specific for tumorigenic processes, the novel tumor marker FEN1 has great potential to be of clinical utility with various classes of tumor types.

Surprisingly, it was found in the present invention that a determination of the concentration of FEN1 in a sample and/or body fluid, allows the assessment of cancer, e.g. of endometrial cancer, malignant melanoma, cervix cancer, head and neck cancer, ovarian cancer, colon cancer, bladder cancer, pancreatic cancer, breast cancer, small cell lung cancer, prostate cancer, kidney cancer or non small cell lung cancer. Even more surprisingly, it was found that a increased concentration of FEN1 or fragments thereof in a sample and/or body fluid compared to normal controls is indicative for the risk or occurrence of cancer.

The present invention relates to a method for assessing cancer in vitro comprising measuring in a serum or plasma sample the concentration of FEN1 by an immunological detection method and using the measurement result, particularly the concentration determined, in the assessment of cancer.

### Summary of the Invention

In one embodiment the present invention relates to a method for assessing cancer in vitro comprising measuring in a serum or plasma sample the concentration of a) Flap endonuclease-1 protein (= FEN1), b) optionally one or more other marker of cancer, and c) using the measurement result of step (a) and optionally of step (b) in the assessment of cancer, wherein a increased concentration of FEN1 protein is indicative for cancer.

Further the present invention relates to the use of FEN1 protein in the in vitro assessment of cancer, wherein an increased concentration of a FEN1 preotein is indicative for cancer, and wherein FEN1 protein is detected in a serum or plasma sample.

Further the present invention relates to the use of an antibody directed against FEN1 in the in vitro assessment of cancer, wherein an increased concentration of FEN1 protein in a serum or plasma sample is indicative for cancer.

Further the present invention discloses the use of a marker panel comprising FEN1 protein and optionally one or more other marker for cancer in the in vitro assessment of cancer, wherein an increased concentration of FEN1 protein in a serum or plasma sample is indicative for cancer.

Surprisingly, it was found that a increased concentration of FEN1 in the test sample is associated with the occurrence of cancer. It could be shown that FEN1 is a marker which is not specific for a single type of cancer, but a marker for different types of cancer, i.e. a general tumor marker. Since FEN1 appears to be rather specific for tumorigenic processes, the novel tumor marker FEN1 has great potential to be of clinical utility with various classes of tumor types.

### Detailed Description of the Invention

The present invention relates to a method for assessing cancer in vitro comprising measuring in a serum or plasma sample the concentration of FEN1 and using the measurement results, particularly the concentration determined in the assessment of cancer.

The method for assessing cancer in vitro comprises measuring in a serum or plasma sample the concentration of (a) FEN1, (b) optionally one or more other marker of cancer, and (c) using the measurement result of step (a) and optionally of step (b) in the assessment of cancer, wherein a increased concentration of FEN1 is indicative for cancer.

The method of the present invention is suitable for the assessment of many different types of cancer. Increased concentrations of FEN1 protein in a serum or plasma sample as compared to normal controls have been found for example in specific cancer types like endometrial cancer, malignant melanoma, cervix cancer, head and neck cancer, ovarian cancer, colon cancer, bladder cancer, pancreatic cancer, breast cancer, small cell lung cancer, prostate cancer, kidney cancer or non small cell lung cancer, respectively.

According to another preferred embodiment of the invention, the concentration of FEN1 protein is measured in a serum or plasma sample in order to assess specific cancer types, such as endometrial cancer (EC), malignant melanoma (MM), cervix cancer (CC), head and neck cancer (H/NC), ovarian cancer (OC), colon cancer (CRC), bladder cancer (BLC), pancreatic cancer (PAC), breast cancer (BC), small cell lung cancer (SCLC), prostate cancer (PC), kidney cancer (KC) or non small cell lung cancer (NSCLC) in vitro.

One embodiment of the present invention refers to the mass screening of a population to distinguish between individuals which are probably free from cancer and individuals which might be classified as "suspect" cases. The latter group of individuals could then be subjected to further diagnostic procedures, e.g. by imaging methods or other suitable means.

A further embodiment of the present invention refers to an improvement of tumor marker panels which are suitable for the diagnosis of cancer in general or tumor marker panels which are suitable for the diagnosis of a specific tumor type.

CYFRA 21-1 is currently regarded to be the best of the presently known tumor markers for lung cancer. Even though not organ-specific it is predominantly found in lung tissue. Sensitivity of CYFRA 21-1 for lung cancer is described to be between 46-61% at a specificity of 95% towards other benign lung diseases. Increased serum levels of CYFRA 21-1 are also associated with pronounced benign liver diseases, renal insufficiency and invasive bladder cancer. CYFRA 21-1 testing is recommended for postoperative therapy surveillance.

CEA belongs to the group of carcinofetal antigens, usually produced during embryogenesis. CEA is not organ-specific and predominantly used for monitoring of colorectal cancer. Besides malignancies, also several benign diseases such as cirrhosis, bronchitis, pancreatitis and autoimmune diseases are associated with increased CEA serum levels. At 95% specificity towards benign lung diseases its sensitivity for lung cancer is reported to be 29-44%. The primary use of CEA is in monitoring colon cancer, especially when the disease has metastasized. However, a variety of cancers can produce elevated levels of CEA, including breast cancer. A preferred use of CEA is therapy surveillance of lung cancer.

NSE is a tumor marker for SCLC. Generally, increased NSE serum levels are found in association with neuroectodermal and neuroendocrine tumors. Increased serum levels are also found in patients with benign lung diseases and cerebral diseases, such as meningitis or other inflammatory diseases of the brain, and traumatic injuries to the head. While sensitivity for SCLC at 95% specificity is reported to be 60-87%, performance of NSE testing for NSCLC is poor (7-25%). NSE is recommended for therapy surveillance of SCLC.

CA 19-9 (carbohydrate antigen 19-9), a sialylated Lewis (a) antigen) on a glycolipid is a tumor marker for gastrointestinal cancers. It occurs in fetal gastric, intestinal and pancreatic epithelia. Low concentrations can also be found in adult tissue in the liver, lungs, and pancreas. There is no correlation between tumor mass and the CA 19-9 assay values Therefore the determination of CA 19-9 cannot be used for the early detection of pancreatic carcinoma. As the mucin is excreted exclusively via the liver, even slight cholestasis can lead to clearly elevated CA 19-9 serum levels in some cases. The marker is mainly used as an aid in the monitoring of disease status in those patients having confirmed pancreatic cancer (sensitivity 70-87%). 3-7% of the population have the Lewis a-negative/b-negative blood group configuration and are unable to express the mucin with the reactive determinant CA 19-9. This must be taken into account when interpreting the findings.

CA 125 is found in a high percentage of non-mucinous ovarian tumors of epithelial origin and can be detected in serum. Ovarian carcinoma accounts for about 20% of gynecological tumors. Although the highest CA 125 values occur in patients suffering from ovarian carcinoma, clearly elevated values are also observed in malignancies of the endometrium, breast, gastrointestinal tract, and various other malignancies. Increased values are sometimes found in various benign gynecological diseases such as ovarian cysts, ovarian metaplasia, endometriosis, uterus myomatosus or cervicitis. Slight elevations of this marker may also occur in early pregnancy and in various benign diseases (e.g. acute and chronic pancreatitis, benign gastrointestinal diseases, renal insufficiency, autoimmune diseases and others). Markedly elevated levels have been found in benign liver diseases such as cirrhosis and hepatitis. Extreme elevations can occur in any kind of ascites due to malignant and benign diseases. Although CA 125 is a relatively unspecific marker, it is today the most important tumor marker for monitoring the therapy and progress of patients with serous ovarian carcinoma. A sensitivity of 69-79% is reported for 82-93% specificity.

PSA ("prostate related antigen") is commonly tested tumor marker used in blood testing. PSA appears to have a high tissue specificity; the glycoprotein is found in normal prostatic epithelium and secretions but not in other tissues. PSA is highly sensitive for the presence of prostatic cancer. The elevation correlated with stage and tumor volume. It is predictive of recurrence and response to treatment. Finally, the antigen has prognostic value in patients with very high values prior to surgery are likely to relapse.

NNMT (nicotinamide N-methyltransferase; Swiss-PROT: P40261) has an apparent molecular weight of 29.6 kDa and an isoelectric point of 5.56. NNMT catalyzes the N-methylation of nicotinamide and other pyridines. This activity is important for biotransformation of many drugs and xenobiotic compounds. The protein has been reported to be predominantly expressed in liver and is located in the cytoplasm. NNMT has been cloned from cDNA from human liver and contained a 792-nucleotide open reading frame that encoded a 264-amino acid protein with a calculated molecular mass of 29.6 kDa (Aksoy, S. et al., J. Biol. Chem. 269 (1994) 14835-14840). Little is known in the literature about a potential role of the enzyme in human cancer. In one paper, increased hepatic NNMT enzymatic activity was reported as a marker for cancer cachexia in mice (Okamura, A. et al., Jpn. J. Cancer Res. 89 (1998) 649-656). In a recent report, down-regulation of the NNMT gene in response to radiation in radiation sensitive cell lines was demonstrated (Kassem, H. et al., Int. J. Cancer 101 (2002) 454-460). It has recently been found (WO 2004/057336) that NNMT will be of interest in the assessment of CRC.

ProGRP is a tumor marker, useful in the detection and monitoring of SCLC. Increased serum levels are also found in patients with nonmalignant lung/pleural diseases, such as idiopathic pulmonary fibrosis or sarcoidosis. While sensitivity for proGRP in the field of SCLC (at 95% specificity) is reported to be 47-86%, the performance of proGRP testing in the field of NSCLC is poor because the sensitivity is reported as being below 10%).

SCC was originally identified in squamous cell CA of the cervix. The sensitivity of SCC for LC in general is low (18-27%). Therefore, SCC testing is regarded to be not suitable for screening. However, due to a higher sensitivity for squamous cell CA, a preferred use for SCC is therapy surveillance, even though CYFRA 21-1 generally performs better.

p53 (TP53, cellular tumor antigen p53, tumor suppressor p53 or phosphoprotein p53) is a transcription factor inducing cell growth arrest or apoptosis (Appella, E. et al., Pathol. Biol. 48 (2000) 227-245). p53 acts as a tumor suppressor in many tumor types and inactivating mutations in its gene are the most common genetic events promoting cancer development in humans (reviewed in Olivier, M. and Petitjean, A., Cancer Gene Ther. 1 (2009) 1-12; Petitjean, A. et al., Oncogene 26 (2007) 2157-2165). p53 mutation is observed in 40-50% of colorectal carcinomas, and is associated with carcinoma aggressiveness (Soussi T., Cancer Res. 60 (2000) 1777-1788). Mutations in p53 gene lead not only to the disruption of the protein function, but also to the expression of tumor-associated antigens (TAA) and initiation of the auto-immune response and generation of specific anti-p53 autoantibodies in sera of cancer patients (Zhang, J.Y. et al., Cancer Epidemiology, Biomarkers & Prevention 12 (2003) 136-143; Soussi T., Cancer Res. 60 (2000) 1777-1788).

Anti-p53 autoantibodies detection in human sera is an emerging tool for the diagnosis and management of cancer. Dependent of the cancer type, the frequency of anti-p53 autoantibodies in sera range from 17.8% (CRC) to 16.1 % (LC) and 7.8% (Breast Cancer) (Tan, E.M. and Zhang, J., Immunological Reviews 222 (2008) 328-340; Zhang, J.Y. et al., Cancer Epidemiology, Biomarkers & Prevention 12 (2003) 136-143).

Seprase, also known as fibroblast activation protein (= FAP), is as a 170 kDa glycoprotein having gelatinase and dipeptidyl peptidase activity consisting of two identical monomeric Seprase units (Pineiro-Sanchez, M.L. et al., J. Biol. Chem. 272 (1997) 7595-7601; Park, J.E. et al., J. Biol. Chem. 274 (1999) 36505-36512). The monomer of the human membrane bound Seprase protein comprises 760 amino acids. Human Seprase is predicted to have its first 4 N-terminal residues within the fibroblast cytoplasm, followed by a 21-residue transmembrane domain and then a 734 residue extracellular C-terminal catalytic domain (Goldstein, L.A. et al., Biochim Biophys Acta. 1361 (1997) 11-19; Scanlan, M.J. et al., Proc Natl Acad Sci USA 91 (1994) 5657-5661). A shorter form of human Seprase protein is known to a person skilled in the art as soluble Seprase or circulating antiplasmin-cleaving enzyme (= APCE) (Lee, K.N. et al., Blood 103 (2004) 3783-3788; Lee, K.N. et al., Blood 107 (2006) 1397-1404), comprising the amino acid positions 26-760 from Swissprot database Accession number Q12884. The dimer of soluble Seprase is a 160 kDa glycoprotein consisting of two identical monomeric soluble Seprase protein units. Piñeiro-Sánchez et al. (supra) found that a increased expression of Seprase correlates with the invasive phenotype of human melanoma and carcinoma cells. Henry, L.R. et al., Clin. Cancer Res. 13 (2007) 1736-1741 describe that human colon tumor patients having high levels of stromal Seprase are more likely to have aggressive disease progression and potential development of metastases or recurrence.

Human dipeptidyl peptidase IV (= DPPIV), which is also known as CD26, is a 110 kDa cell surface molecule. The amino acid sequence of human DPPIV protein comprises 766 amino acids. It contains intrinsic dipeptidyl peptidase IV activity which selectively removes N-terminal dipeptide from peptides with proline or alanine in the third amino acid position. It interacts with various extracellular molecules and is also involved in intracellular signal transduction cascades. The multifunctional activities of human DPPIV are dependent on cell type and intracellular or extracellular conditions that influence its role as a proteolytic enzyme, cell surface receptor, co-stimulatory interacting protein and signal transduction mediator. Human DPPIV has a short cytoplasmatic domain from amino acid position 1 to 6, a transmembrane region from amino acid position 7 to 28, and an extracellular domain from amino acid position 29 to 766 with intrinsic dipeptidyl peptidase IV (DPPIV) activity. Human soluble dipeptidyl peptidase IV (= soluble DPPIV) comprises the amino acid positions 29 to 766 from Swissprot database Accession number P27487. The dimer of soluble DPPIV is a 170 kDa glycoprotein consisting of two identical monomeric soluble DPPIV units.

Soluble DPPIV/Seprase complex (= DPPIV/Seprase) refers to the soluble complex formed of a soluble DPPIV homodimer (170 kDa) and a soluble Seprase homodimer (160 kDa) with a molecular weight of 330 kDa. Under certain conditions this complex may form a double complex having a molecular weight of 660 kDa.

The present invention is also directed to a method for assessing cancer in vitro by biochemical markers, comprising measuring in a serum or plasma sample the concentration of FEN1 protein and of one or more other markers specific for cancer, and using the measurement results, particularly the concentrations, determined in the assessment of cancer. Preferred markers for use in combination with FEN1 are, on the one hand, markers which are general tumor markers (i.e. markers which are not specific for a single tumor type) or, on the other hand, specific tumor markers (markers which are specific for a single tumor type). Preferred markers, e.g. for the assessment of cancer are Cyfra 21-1, CEA, NSE, CA 19-9, CA 125, PSA, proGRP, SCC, NNMT, anti-p53 autoantibodies, Seprase and soluble DPPIV/Seprase complex (= DPPIV/Seprase). These markers may be used individually each or in any combination together with FEN1.

The present invention is also directed to a method for assessing cancer in vitro by biochemical markers, comprising measuring in a serum or plasma sample the concentration of FEN1 and of one or more other cancer markers and using the measurement results, particularly concentrations determined in the assessment of cancer. It is preferred that the one or more other marker is selected from the group consisting of Cyfra 21-1, CEA, NSE, CA 19-9, CA 125, PSA, proGRP, SCC, NNMT, anti-p53 autoantibodies, Seprase and DPPIV/Seprase.

The present invention is also directed to the use of a marker panel comprising at least the marker FEN1 and at least one other tumor marker(s), selected from the group consisting of Cyfra 21-1, CEA, NSE, CA 19-9, CA 125, PSA, proGRP, SCC, NNMT, anti-p53 autoantibodies, Seprase and DPPIV/Seprase, in the assessment of cancer.

Preferably, the present invention is directed to a method for assessing cancer in vitro by biochemical markers, comprising measuring in a serum or plasma sample the concentration of FEN1 and of one or more other cancer markers and using the measurement results, particularly concentrations determined in the assessment of cancer. It is preferred that the one or more other marker is selected from the group consisting of Cyfra 21-1, CEA, NSE, CA 19-9, CA 125, PSA, proGRP, SCC, NNMT, anti-p53 autoantibodies, Seprase and DPPIV/Seprase.

The present invention also relates to the use of FEN1 protein in the assessment of cancer, wherein a increased concentration of FEN1 is indicative for cancer.

The present invention also relates to the use of FEN1 protein in the assessment of cancer in vitro, wherein the sample is serum or plasma.

The present invention also relates to the use of FEN1 in the assessment of several specific types of cancer, particularly EC, MM, CC, H/NC, OC, CRC, BLC, PAC, BC, SCLC, PC, KC or NSCLC.

The present invention also relates to the use of an antibody directed against FEN1 protein in the assessment of cancer, wherein a increased concentration of FEN1 in a serum or plasma sample is indicative for cancer.

Preferably FEN1 is detected in a sandwich-type immunoassay format (= sandwich immunoassay).

The term "measurement" preferably comprises a qualitative, semi-quanitative or a quantitative measurement of FEN1 protein in a sample. In a preferred embodiment the measurement is a semi-quantitative measurement, i.e. it is determined whether the concentration of FEN1 is above or below a cut-off value. As the skilled artisan will appreciate, in a Yes- (presence) or No- (absence) assay, the assay sensitivity is usually set to match the cut-off value. A cut-off value can for example be determined from the testing of a group of healthy individuals. Preferably the cut-off is set to result in a specificity of 90%, also preferred the cut-off is set to result in a specificity of 95%, or also preferred the cut-off is set to result in a specificity of 98%. A value above the cut-off value can for example be indicative for the presence of cancer. In particular a value above the cut-off value can for example be indicative for the presence of EC, MM, CC, H/NC, OC, CRC, BLC, PAC, BC, SCLC, PC, KC and/or NSCLC. In a further preferred embodiment the measurement of FEN1 is a quantitative measurement. In further embodiments the concentration of FEN1 is correlated to an underlying diagnostic question like e.g. stage of disease, disease progression, or response to therapy.

In another preferred embodiment, the cut-off is set to result in a sensitivity of 90%, also preferred the cut-off is set to result in a sensitivity of 95%, or also preferred the cut-off is set to result in a sensitivity of 98%.

A value below the cut-off value can for example be indicative for the absence of cancer. In particular a value below the cut-off value can for example be indicative for the absence of EC, MM, CC, H/NC, OC, CRC, BLC, PAC, BC, SCLC, PC, KC and/or NSCLC.

In a further preferred embodiment the measurement of FEN1 is a quantitative measurement. In further embodiments the concentration of FEN1 is correlated to an underlying diagnostic question like e.g. stage of disease, disease progression, or response to therapy.

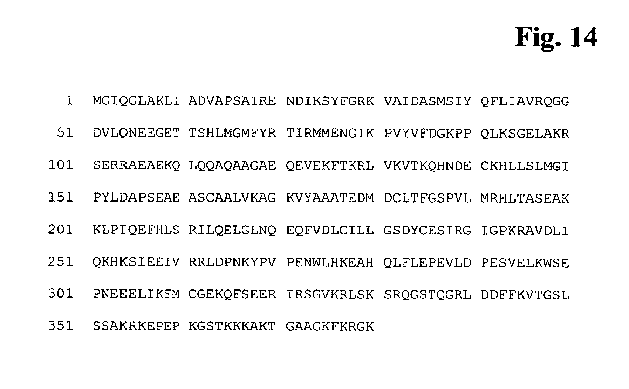

Flap endonuclease-1 protein (= FEN1), Swiss-PROT ID: P39748, is a nuclear protein of 380 amino acids with a molecular weight of 42.6 kDa, characterized by the sequence given in SEQ ID NO:1 (Fig. 14). The coding sequence of human FEN1 was predicted by Murray in 1994 (Murray J.M. et al., Mol. Cell. Biol. 14 (1994) 4878-4888) from a newly cloned sequence. Based on the function of the yeast homolog rad2 a function in high fidelity chromosome segregation and in the repair of UV-induced DNA damage was suggested. As these are fundamental processes in chromosomal integrity, the authors also proposed an involvement of the protein in cancer avoidance. The gene locus on human chromosome 11 was later identified by Hiraoka et al. (Hiraoka L.R. et al., Genomics 25 (1995) 220-225) and Taylor et al. (Taylor T.D. et al., Nature 440 (2006) 497-500). The functions of FEN1 and its interactions with DNA have been the focus of numerous studies (Robins P. et al., J. Biol. Chem. 269 (1994) 28535-28538), Shen B. et al., J. Biol. Chem. 271 (1996) 9173-9176, Hasan S. et al., Mol. Cell 7 (2001) 1221-1231, Qiu J. et al., J. Biol. Chem. 277 (2002) 24659-24666 and Sakurai S. et al, EMBO J. 24 (2005) 683-693). Several enzymatic functions in DNA metabolism have been demonstrated including endonuclease activity that cleaves the 5'-overhanging flap structure generated by displacement synthesis when DNA polymerase encounters the 5'-end of a downstream Okazaki fragment. Additionally FEN1 also possesses a 5' to 3' exonuclease activity on niked or gapped double-stranded DNA, and exhibits RNase H activity. These have been reviewed by Shen et al. (Shen B. et al. BioEssays 27 (2005) 717-729) or Liu et al. (Liu Y. et al., Annu. Rev. Biochem. 73 (2004) 589-615).

Only recently mutations, deregulated expression and functional defects of FEN1 have been shown to be involved in the development of diseases. Kucherlapati et al. (Kucherlapati M. et al., PNAS 99 (2002) 9924-9929) linked FEN1 expression to tumor progression. Sato et al (Sato M. et al., Oncogene 22 (2003) 7243-7246) have demonstrated an increased expression of FEN1 in lung cancer while Lam et al (Lam J.S. et al., BJU International 98 (2006) 445-451) found overexpression in prostate cancer. Singh et al. (Singh P. et al., Mol. Cancer Res. 6 (2008) 1710-1717) found overexpression of FEN1 in breast cancer and other cancers that was regulated by hypomethylation. A much broader link to disease processes was published by Zheng et al. (Zheng L. et al., Nature medicine 13 (2007) 812-819) where the authors could show that FEN1 mutations may lead to autoimmunity, chronic inflammation and cancer.

Though the involvement of FEN1 in diseases has now clearly been established, studies on FEN1 protein as a biomarker of these diseases have not been published. So far available studies focus on DNA or RNA-based methods. This is in line with patents on FEN1 (WO 2008/089577: Breast cancer gene array, WO 2008/151110: Molecular diagnosis and typing of lung cancer variants, WO2008/077165: Set of tumor markers, WO 2007/073220: Prognosis prediction for colorectal cancer, US 5,874,283: Mammalian flap-specific endonuclease).

As used herein, each of the following terms has the meaning associated with it in this section.

The articles "a" and "an" are used herein to refer to one or to more than one (i.e. to at least one) of the grammatical object of the article. By way of example, "a marker" means one marker or more than one marker. The term "at least" is used to indicate that optionally one or more further objects may be present. By way of example, a marker panel comprising at least (the markers) FEN1 and CYFRA 21-1 may optionally comprise one or more other marker.

The expression "one or more" denotes 1 to 50, preferably 1 to 20 also preferred 2, 3, 4, 5, 6, 7, 8, 9, 10, 12, or 15.

The terms "chip", "bio-chip", "polymer-chip" or "protein-chip" are used interchangeably and refer to a collection of a large number of probes, markers or biochemical markers arranged on a shared substrate which could be a portion of a silicon wafer, a nylon strip, a plastic strip, or a glass slide.

An "array," "macroarray" or "microarray" is an intentionally created collection of substances, such as molecules, markers, openings, microcoils, detectors and/or sensors, attached to or fabricated on a substrate or solid surface, such as glass, plastic, silicon chip or other material forming an array. The arrays can be used to measure the levels of large numbers, e.g., tens, thousands or millions, of reactions or combinations simultaneously. An array may also contain a small number of substances, e.g., one, a few or a dozen. The substances in the array can be identical or different from each other. The array can assume a variety of formats, e.g., libraries of soluble molecules, libraries of immobilized molecules, libraries of immobilized antibodies, libraries of compounds tethered to resin beads, silica chips, or other solid supports. The array could either be a macroarray or a microarray, depending on the size of the pads on the array. A macroarray generally contains pad sizes of about 300 microns or larger and can be easily imaged by gel and blot scanners. A microarray would generally contain pad sizes of less than 300 microns.

A "solid support" is insoluble, functionalized, polymeric material to which library members or reagents may be attached or covalently bound (often via a linker) to be immobilized or allowing them to be readily separated (by filtration, centrifugation, washing etc.) from excess reagents, soluble reaction by- products, or solvents.

The term "marker" or "biochemical marker" as used herein refers to a molecule to be used as a target for analyzing a patient's test sample. Examples of such molecular targets are proteins or polypeptides. Proteins or polypeptides used as a marker in the present invention are contemplated to include naturally occurring variants of said protein as well as fragments of said protein or said variant, in particular, immunologically detectable fragments. Immunologically detectable fragments preferably comprise at least 6, 7, 8, 10, 12, 15 or 20 contiguous amino acids of said marker polypeptide. One of skill in the art would recognize that proteins which are released by cells or present in the extracellular matrix may be damaged, e.g., during inflammation, and could become degraded or cleaved into such fragments. Certain markers are synthesized in an inactive form, which may be subsequently activated by proteolysis. As the skilled artisan will appreciate, proteins or fragments thereof may also be present as part of a complex. Such complex also may be used as a marker in the sense of the present invention. Variants of a marker polypeptide are encoded by the same gene, but may differ in their isoelectric point (=PI) or molecular weight (=MW), or both e.g., as a result of alternative mRNA or pre-mRNA processing. The amino acid sequence of a variant is to 95% or more identical to the corresponding marker sequence. In addition, or in the alternative a marker polypeptide or a variant thereof may carry a posttranslational modification. Non-limiting examples for posttranslational modifications are glycosylation, acylation, and/or phosphorylation.

FEN1 proteins, particularly soluble forms of FEN1 proteins and/or fragments thereof, are detected in appropriate samples. Preferred samples are tissue samples, tissue lysates or body fluids, such as blood, plasma, serum, urine, bronchioalveolar lavage (= BAL; preferred in the case of suspected lung cancer (LC)) or epithelial lining fluid (= ELF; preferred in the case of suspected LC). Preferably, the sample is derived from a human subject, e.g. a tumor patient or a person in risk of a tumor or a person suspected of having a tumor. Also preferred FEN1 is detected in a serum or plasma sample.

In a preferred embodiment according to the present invention, the concentration of FEN1 protein is determined. In one embodiment, the marker FEN1 is specifically measured from a sample by use of a specific binding agent.

A specific binding agent is, e.g., a receptor for FEN1, a lectin binding to FEN1 or an antibody reactive with FEN1. A specific binding agent has at least an affinity of 10⁷ l/mol for its corresponding target molecule. The specific binding agent preferably has an affinity of 10⁸ l/mol or also preferred of 10⁹ l/mol for its target molecule.

As the skilled artisan will appreciate the term specific is used to indicate that other biomolecules present in the sample do not significantly bind to the binding agent specific for FEN1. Preferably, the level of binding to a biomolecule other than the target molecule results in a binding affinity which is at most only 10% or less, only 5% or less only 2% or less or only 1% or less of the affinity to the target molecule, respectively. A preferred specific binding agent will fulfil both the above minimum criteria for affinity as well as for specificity.

A specific binding agent preferably is an antibody reactive with FEN1. The term antibody refers to a polyclonal antibody, a monoclonal antibody, antigen binding fragments of such antibodies, single chain antibodies as well as to genetic constructs comprising the binding domain of an antibody.

Any antibody fragment retaining the above criteria of a specific binding agent can be used. Antibodies are generated by state of the art procedures, e.g., as described in Tijssen (Tijssen, P., Practice and theory of enzyme immunoassays, 11, Elsevier Science Publishers B.V., Amsterdam, the whole book, especially pages 43-78). In addition, the skilled artisan is well aware of methods based on immunosorbents that can be used for the specific isolation of antibodies. By these means the quality of polyclonal antibodies and hence their performance in immunoassays can be enhanced (Tijssen, P., supra, pages 108-115).

For the achievements as disclosed in the present invention polyclonal antibodies raised in rabbits may be used. However, clearly also polyclonal antibodies from different species, e.g., sheep or goat, as well as monoclonal antibodies can also be used. Since monoclonal antibodies can be produced in any amount required with constant properties, they represent ideal tools in development of an assay for clinical routine. The generation and the use of monoclonal antibodies to FEN1 in a method according to the present invention, respectively, represent yet other preferred embodiments.

Immunoassays are well known to the skilled artisan. Methods for carrying out such assays as well as practical applications and procedures are summarized in related textbooks. Examples of related textbooks are Tijssen, P., Preparation of enzyme-antibody or other enzyme-macromolecule conjugates, In: Practice and theory of enzyme immunoassays, pp. 221-278, Burdon, R.H. and v. Knippenberg, P.H. (eds.), Elsevier, Amsterdam (1990), and various volumes of Methods in Enzymology, Colowick, S.P., and Caplan, N.O. (eds.), Academic Press), dealing with immunological detection methods, especially volumes 70, 73, 74, 84, 92 and 121.

As the skilled artisan will appreciate now that FEN1 has been identified as a marker which is useful in the assessment of cancer, preferably lung cancer, various immunodiagnostic procedures may be used to reach a result comparable to the achievements of the present invention. For example, alternative strategies to generate antibodies may be used. Such strategies comprise amongst others the use of synthetic peptides, representing an epitope of FEN1 for immunization. Alternatively, DNA immunization also known as DNA vaccination may be used.

For measurement the sample obtained from an individual is incubated with the specific binding agent for FEN1 under conditions appropriate for formation of a binding agent FEN1 complex. Such conditions need not be specified, since the skilled artisan without any inventive effort can easily identify such appropriate incubation conditions. The amount of binding agent FEN1 complex is measured and used in the assessment of cancer, preferably of lung cancer. As the skilled artisan will appreciate there are numerous methods to measure the amount of the specific binding agent FEN1 complex all described in detail in relevant textbooks (cf., e.g., Tijssen P., supra, or Diamandis, E.P. and Christopoulos, T.K. (eds.), Immunoassay, Academic Press, Boston (1996)).

Preferably FEN1 is detected in a sandwich-type assay format (= sandwich immunoassay). In such sandwich immunoassay, a first specific binding agent attached to a solid support is used to capture FEN1 on the one side and a second specific binding agent, which is labeled to be directly or indirectly detectable, is used on the other side. The specific binding agents used in a sandwich-type assay format may be a combination of antibodies specifically directed against FEN 1.

A "marker of cancer" in the sense of the present invention is any marker that if combined with the marker FEN1 adds relevant information in the assessment of cancer disease in the assessment of cancer in general or in the assessment of certain cancer types, e.g. in the assessment of EC, MM, CC, H/NC, OC, CRC, BLC, PAC, BC, SCLC, PC, KC or NSCLC. The information is considered relevant or of additive value if at a given specificity the sensitivity, or if at a given sensitivity the specificity, respectively, for the assessment of cancer can be improved by including said marker into a marker combination already comprising the marker FEN1. In the preferred embodiment of cancer assessment, the improvement in sensitivity or specificity, respectively, is statistically significant at a level of significance of p = .05, .02, .01 or lower. Preferably, the one or more other tumor marker is selected from the group consisting of Cyfra 21-1, CEA, NSE, CA 19-9, CA 125, PSA, proGRP, SCC, NNMT, anti-p53 autoantibodies, Seprase and DPPIV/Seprase.

The term "sample" as used herein refers to a biological sample obtained for the purpose of evaluation in vitro. In the methods of the present invention, the sample or patient sample preferably may comprise any body fluid. Preferred samples are tissue samples, tissue lysates or body fluids, such as whole blood, serum, plasma, urine, bronchioalveolar lavage (= BAL; preferred in the case of suspected lung cancer (LC)) or epithelial lining fluid (= ELF; preferred in the case of suspected LC), with serum or plasma being most preferred.

The term "tissue sample" and/or "tissue section" as used herein refers to a biological sample taken from a patient during surgery, therapeutic resections or a biopsy (e.g. incisional biopsy, excisional biopsy, core biopsy or needle aspiration biopsy) involving the removal of cells or tissues for the purpose of evaluation in vitro. When performing an analysis according to the present invention, the tissue sample material is used either directly or as a "tissue lysate". A "tissue sample" as used herein refers also to thin tissue slices usually accomplished through the use of a microtome. In any disclosed method embodiment involving a biological sample, such biological sample can be (but is not necessarily) mounted on a microscope slide, is a tissue section (such as a formalin-fixed and paraffin-embedded tissue section), and/or is a neoplastic tissue (such as, a lung cancer, colorectal cancer, head and neck cancer, gastric cancer, or glioblastoma).

A "tissue lysate", "cell lysate", "lysate", "lysed sample", "tissue extract" or "cell extract" as used herein refers to a sample and/or biological sample material comprising lysed tissue or cells, i.e. wherein the structural integrity of tissue or cells has been disrupted. To release the contents of cells or a tissue sample, the material is usually treated with enzymes and/or with chemicals to dissolve, degrade or disrupt the cellular walls and cellular membranes of such tissues or cells. The skilled artisan is fully familiar with appropriate methods for obtaining lysates. This process is encompassed by the term "lysis".

The term "assessing cancer" and in particular "assessing endometrial cancer (EC), malignant melanoma (MM), cervix cancer (CC), head and neck cancer (H/NC), ovarian cancer (OC), colon cancer (CRC), bladder cancer (BLC), pancreatic cancer (PAC), breast cancer (BC), small cell lung cancer (SCLC), prostate cancer (PC), kidney cancer (KC) or non small cell lung cancer (NSCLC)" is used to indicate that the method according to the present invention will (alone or together with other markers or variables, e.g., the criteria set forth by the UICC (see above)) e.g., aid the physician to establish or confirm the absence or presence of cancer or aid the physician in the prognosis, the detection of recurrence (follow-up of patients after surgery) and/or the monitoring of treatment, especially of chemotherapy.

As the skilled artisan will appreciate, any such assessment is made in vitro. The patient sample is discarded afterwards. The patient sample is solely used for the in vitro diagnostic method of the invention and the material of the patient sample is not transferred back into the patient's body. Typically, the sample is a liquid sample, e.g., whole blood, serum, or plasma.

Unless otherwise noted, technical terms are used according to conventional usage. Definitions of common terms in cell and molecular biology may be found in Lewin, B., Genes V, published by Oxford University Press (1994), ISBN 0-19-854287 9); Kendrew, J. et al. (eds.), The Encyclopedia of Molecular Biology, published by Blackwell Science Ltd. (1994), ISBN 0-632-02182-9); and Meyers, R.A. (ed.), Molecular Biology and Biotechnology: a Comprehensive Desk Reference, published by VCH Publishers, Inc. (1995), ISBN 1-56081-569 8).

In a preferred embodiment the present invention relates to a method for assessing cancer in vitro by biochemical markers, comprising measuring in a sample the concentration of FEN1 and using the concentration determined in the assessment of cancer.

The inventors of the present invention have surprisingly been able to detect a increased concentration of the marker FEN1 in a significant percentage of samples derived from patients with cancer, in particular with EC, MM, CC, H/NC, OC, CRC, BLC, PAC, BC, SCLC, PC, KC or NSCLC. Even more surprising they have been able to demonstrate that the increased concentration of FEN1 in such sample obtained from an individual can be used in the assessment of cancer, in particular of the above-mentioned cancer diseases.

The ideal scenario for diagnosis would be a situation wherein a single event or process would cause the respective disease as, e.g., in infectious diseases. In all other cases correct diagnosis can be very difficult, especially when the etiology of the disease is not fully understood as is the case for many cancer types. As the skilled artisan will appreciate, no biochemical marker is diagnostic with 100% specificity and at the same time 100% sensitivity for a given multifactorial disease. Rather, biochemical markers, e.g., Cyfra 21-1, CEA, NSE, or as shown here FEN1 can be used to assess with a certain likelihood or predictive value e.g., the presence, absence, or the severity of a disease. Therefore in routine clinical diagnosis, generally various clinical symptoms and biological markers are considered together in the diagnosis, treatment and management of the underlying disease.

Biochemical markers can either be determined individually or in a preferred embodiment of the invention they can be measured simultaneously using a chip or a bead based array technology. The concentrations of the biomarkers are then either interpreted independently, e.g., using an individual cut-off for each marker, or they are combined for interpretation.

In a further preferred embodiment the assessment of cancer according to the present invention is performed in a method comprising measuring in a serum or plasma sample the concentration of a) FEN1 protein, b) one or more other marker of cancer, and c) using the measurement result, e.g. the concentration determined in step (a) and step (b), respectively, in the assessment of cancer.

In the assessment of cancer the marker FEN1 will be of advantage in one or more of the following aspects: screening; diagnostic aid; prognosis; monitoring of therapy such as chemotherapy, radiotherapy, and immunotherapy.

### Screening:

Screening is defined as the systematic application of a test to identify individuals e.g. at risk individuals, for indicators of a disease, e.g., the presence of cancer. Preferably the screening population is composed of individuals known to be at higher than average risk of cancer. For example, a screening population for lung cancer is composed of individuals known to be at higher than average risk of lung cancer, like smokers, ex-smokers, and uranium-, quartz- or asbestos-exposed workers.

In the preferred embodiment any body fluid such as whole blood, plasma or serum is used as a sample in the screening for cancer.

For many diseases, no single biochemical marker in the circulation will ever meet the sensitivity and specificity criteria required for screening purposes. This appears to be also true for cancer and in particular for lung cancer. It has to be expected that a marker panel comprising a plurality of markers will have to be used in cancer screening. The data established in the present invention indicate that the marker FEN1 will form an integral part of a marker panel appropriate for screening purposes. The present invention therefore relates to the use of FEN1 as one marker of a cancer marker panel, i.e. a marker panel comprising FEN1 and one or more additional marker for cancer screening purposes. In particular, the present invention relates to the use of FEN1 as one marker of a general cancer marker panel. Such marker panel comprises the marker FEN1 and one or more additional markers, e.g. general cancer markers and/or markers for the above-mentioned type of cancer.

A combination of markers significantly improves the value of the molecular assay. First, the sensitivity of the assay is significantly improved using the marker panel. Second, sophisticated statistical models permit ROC curve analysis of the multi marker assay, and the results confirm that the diagnostic accuracy is significantly increased compared to the best individual marker.

FEN1 is also likely to contribute to marker panels for certain specific types of cancer, e.g. EC, MM, CC, H/NC, OC, CRC, BLC, PAC, BC, SCLC, PC, KC or NSCLC.

The present data further indicate that certain combinations of markers will be advantageous in the screening for cancer.

For example, with reference to the preferred embodiment of screening cancer, the present invention also relates to the use of a marker panel comprising FEN1 and at least one or more marker(s) selected from the group consisting of Cyfra 21-1, CEA, NSE, CA 19-9, CA 125, PSA, proGRP, SCC, NNMT, anti-p53 autoantibodies, Seprase and DPPIV/Seprase.

### Diagnostic aid:

Markers may either aid the differential diagnosis of benign vs. malignant disease in a particular organ, help to distinguish between different histological types of a tumor, or to establish baseline marker values before surgery.

Today, important methods used in the detection of lung cancer are radiology and/or computed tomography (CT) scans. Small nodules, i.e. small regions of suspect tissue can be visualized by these methods. However, many of these nodules - more than 90% with CT - represent benign tissues changes, and only a minority of nodules represents cancerous tissue. Use of the marker FEN1 may aid in the differentiation of benign versus malign disease.

In a preferred embodiment the marker FEN1 is used in an immunohistological method in order to establish or confirm different histological types of cancer.

Since FEN1 as a single marker might be superior to other markers, e.g. to other markers, like CEA or NSE, it has to be expected that FEN1 will be used as a diagnostic aid, especially by establishing a baseline value before surgery. The present invention thus also relates to the use of FEN1 for establishing a baseline value before surgery for cancer.

### Prognosis:

Prognostic indicators can be defined as clinical, pathological, or biochemical features of cancer patients and their tumors that predict with a certain likelihood the disease outcome. Their main use is to help to rationally plan patient management, i.e. to avoid undertreatment of aggressive disease and overtreatment of indolent disease, respectively. Molina, R. et al., Tumor Biol. 24 (2003) 209-218 evaluated the prognostic value of CEA, CA 125, CYFRA 21-1, SSC and NSE in NSCLC. In their study abnormal serum levels of the markers NSE, CEA, and LDH (lactate dehydrogenase) appeared to indicate shorter survival.

As FEN1 alone significantly contributes to the differentiation of cancer patients from healthy controls, it has to be expected that it will aid in assessing the prognosis of patients suffering from cancer. The level of preoperative FEN1 will most likely be combined with one or more other marker for cancer and/or the TNM staging system.

### Monitoring of Chemotherapy:

Merle, P. et al., Int. J. of Biological Markers 19 (2004) 310-315 have evaluated CYFRA 21-1 serum level variations in patients with locally advanced NSCLC treated with induction chemotherapy. They conclude that early monitoring of CYFRA 21-1 serum levels may be a useful prognostic tool for tumor response and survival in stage III NSCLC patients. In addition, reports have described the use of CEA in monitoring the treatment of patients with LC (Fukasawa, T. et al., Gan to Kagaku Ryoho 13 (1986) 1862-1867) Most of these were retrospective, non-randomized and contained small numbers of patients. As in the case of the studies with CYFRA 21-1 the CEA studies suggested: a) that patients with a decrease in CEA levels while receiving chemotherapy generally had a better outcome than those patients whose CEA levels failed to decrease and (b) for almost all patients, increases in CEA levels were associated with disease progression.

It is expected that FEN1 will be at least as good a marker for monitoring of chemotherapy as CYFRA 21-1 or CEA, respectively. The present invention therefore also relates to the use of FEN1 in the monitoring of cancer patients under therapy.

In the monitoring of therapy in one preferred embodiment the measurements for FEN1 and for at least one marker selected from the group consisting of Cyfra 21-1, CEA, NSE, CA 19-9, CA 125, PSA, proGRP, SCC, NNMT, anti-p53 autoantibodies, Seprase and DPPIV/Seprase will be combined and used in the assessment of cancer.

### Follow-up:

A large portion of LC patients who undergo surgical resection aimed at complete removal of cancerous tissue, later develop recurrent or metastatic disease (Wagner, H. Jr., Chest 117 (2000) S110-S118; Buccheri, G. et al., Ann. Thorac. Surg. 75 (2003) 973-980). Most of these relapses occur within the first 2-3 years after surgery. Since recurrent/metastatic disease is invariably fatal if detected too late, considerable research has focused on cancer relapse at an early and thus potentially treatable stage.

Consequently, many cancer patients undergo a postoperative surveillance program which frequently includes regular monitoring with CEA. Serial monitoring with CEA one year after surgical resection has been shown to detect an early postoperative recurrent/metastatic disease with a sensitivity of approximately 29 %, at a specificity of approximately 97 %, even in the absence of suspicious symptoms or signs (Buccheri, G. et al., Ann. Thorac. Surg. 75 (2003) 973-980). Thus, the follow-up of patients with cancer after surgery is one of the most important fields of use for an appropriate biochemical marker. Due to the high sensitivity of FEN1 in the cancer patients investigated it is likely that FEN1 alone or in combination with one or more other marker will be of great help in the follow-up of cancer patients after surgery. The use of a marker panel comprising FEN1 and one or more other marker of cancerin the follow-up of cancer patients represents a further preferred embodiment of the present invention.

In yet a further prefererred embodiment the present invention relates to the use of FEN1 in the diagnostic field of cancer.

In yet a further preferred embodiment the present invention relates to the use of FEN1 as a marker molecule for cancer, e.g. for cancer in general or for specific types of cancer, such as EC, NN, CC, H/NC, OC, CRC, BLC, PAC, BC, SCLC, PC, KC or NSCLC in combination with one or more further marker molecules for cancer. The further marker molecules may be cancer-type unspecific general marker molecules and/or cancer-type specific marker molecules. FEN1 and the at least one further marker are used in the assessment of cancer in a liquid sample obtained from an individual. Preferred selected other cancer markers with which the measurement of FEN1 may be combined are Cyfra 21-1, CEA, NSE, CA 19-9, CA 125, PSA, proGRP, SCC, NNMT, anti-p53 autoantibodies, Seprase and DPPIV/Seprase.

As the skilled artisan will appreciate there are many ways to use the measurements of two or more markers in order to improve the diagnostic question under investigation. In a quite simple, but nonetheless often effective approach, a positive result is assumed if a sample is positive for at least one of the markers investigated. This may e.g. the case when diagnosing an infectious disease, like AIDS.

Frequently, however, the combination of markers is evaluated. Preferably the values measured for markers of a marker panel, e.g. for FEN1 and CYFRA 21-1, are mathematically combined and the combined value is correlated to the underlying diagnostic question. Marker values may be combined by any appropriate state of the art mathematical method. Well-known mathematical methods for correlating a marker combination to a disease employ methods like, discriminant analysis (DA) (i.e. linear-, quadratic-, regularized-DA), Kernel Methods (i.e. SVM), Nonparametric Methods (i.e. k-Nearest-Neighbor Classifiers), PLS (Partial Least Squares), Tree-Based Methods (i.e. Logic Regression, CART, Random Forest Methods, Boosting/Bagging Methods), Generalized Linear Models (i.e. Logistic Regression), Principal Components based Methods (i.e. SIMCA), Generalized Additive Models, Fuzzy Logic based Methods, Neural Networks and Genetic Algorithms based Methods. The skilled artisan will have no problem in selecting an appropriate method to evaluate a marker combination of the present invention. Preferably the method used in correlating the marker combination of the invention e.g. to the absence or presence of LC is selected from DA (i.e. Linear-, Quadratic-, Regularized Discriminant Analysis), Kernel Methods (i.e. SVM), Nonparametric Methods (i.e. k-Nearest-Neighbor Classifiers), PLS (Partial Least Squares), Tree-Based Methods (i.e. Logic Regression, CART, Random Forest Methods, Boosting Methods), or Generalized Linear Models (i.e. Logistic Regression). Details relating to these statistical methods are found in the following references: Ruczinski, I., et al, J. of Computational and Graphical Statistics, 12 (2003) 475-511; Friedman, J. H., J. of the American Statistical Association 84 (1989) 165-175; Hastie, T. et al., The Elements of Statistical Learning, Springer Series in Statistics (2001); Breiman, L., et al., Classification and regression trees, California: Wadsworth (1984); Breiman, L., Random Forests, Machine Learning, 45 (2001) 5-32; Pepe, M.S., The Statistical Evaluation of Medical Tests for Classification and Prediction, Oxford Statistical Science Series, 28 (2003); and Duda, R.O. et al., Pattern Classification, Wiley Interscience, 2nd edition (2001).

It is a preferred embodiment of the invention to use an optimized multivariate cut-off for the underlying combination of biological markers and to discriminate state A from state B, e.g. diseased from healthy. In this type of analysis the markers are no longer independent but form a marker panel.

Accuracy of a diagnostic method is best described by its receiver-operating characteristics (ROC) (see especially Zweig, M.H., and Campbell, G., Clin. Chem. 39 (1993) 561-577). The ROC graph is a plot of all of the sensitivity/specificity pairs resulting from continuously varying the decision thresh-hold over the entire range of data observed.

The clinical performance of a laboratory test depends on its diagnostic accuracy, or the ability to correctly classify subjects into clinically relevant subgroups. Diagnostic accuracy measures the test's ability to correctly distinguish two different conditions of the subjects investigated. Such conditions are for example health and disease or benign versus malignant disease.

In each case, the ROC plot depicts the overlap between the two distributions by plotting the sensitivity versus 1 - specificity for the complete range of decision thresholds. On the y-axis is sensitivity, or the true-positive fraction [defined as (number of true-positive test results)/(number of true-positive + number of false-negative test results)]. This has also been referred to as positivity in the presence of a disease or condition. It is calculated solely from the affected subgroup. On the x-axis is the false-positive fraction, or 1 - specificity [defined as (number of false-positive results)/(number of true-negative + number of false-positive results)]. It is an index of specificity and is calculated entirely from the unaffected subgroup. Because the true- and false-positive fractions are calculated entirely separately, by using the test results from two different subgroups, the ROC plot is independent of the prevalence of disease in the sample. Each point on the ROC plot represents a sensitivity/I-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. (If the ROC plot falls completely below the 45° diagonal, this is easily remedied by reversing the criterion for "positivity" from "greater than" to "less than" or vice versa.) Qualitatively, the closer the plot is to the upper left corner, the higher the overall accuracy of the test.

One preferred way to quantify the diagnostic accuracy of a laboratory test is to express its performance by a single number. Such an overall parameter e.g. is the so-called "total error" or alternatively the "area under the curve = AUC". The most common global measure is the area under the ROC plot. By convention, this area is always ≥ 0.5 (if it is not, one can reverse the decision rule to make it so). Values range between 1.0 (perfect separation of the test values of the two groups) and 0.5 (no apparent distributional difference between the two groups of test values). The area does not depend only on a particular portion of the plot such as the point closest to the diagonal or the sensitivity at 90% specificity, but on the entire plot. This is a quantitative, descriptive expression of how close the ROC plot is to the perfect one (area = 1.0).

Combining measurements of FEN1 with other markers like CYFRA 21-1 or CEA, or with other markers of cancer yet to be discovered, FEN1 leads and will lead, respectively, to further improvements in assessment of cancer.

In a preferred embodiment the present invention relates to a method for improving the diagnostic accuracy for cancer versus healthy controls by measuring in a sample the concentration of at least FEN1 and one or more other tumor markers selected from the group consisting of CYFRA 21-1, CEA, NSE, CA 19-9, CA 125, PSA, proGRP, SCC, NNMT, anti-p53 autoantibodies, Seprase and DPPIV/Seprase, respectively and correlating the concentrations determined to the presence or absence of cancer, the improvement resulting in more patients being correctly classified as suffering from cancer versus healthy controls as compared to a classification based on any single marker investigated alone.

In a further preferred embodiment the present invention relates to a method for improving the diagnostic accuracy for cancer versus healthy controls by measuring in a sample the concentration of at least FEN1 and Cyfra 21-1, and optionally of CEA and/or NSE, respectively and correlating the concentrations determined to the presence or absence of cancer, the improvement resulting in more patients being correctly classified as suffering from cancer versus healthy controls as compared to a classification based on any single marker investigated alone.

The following examples, figures, and the sequence listing are provided to aid the understanding of the present invention, the true scope of which is set forth in the appended claims. It is understood that modifications can be made in the procedures set forth without departing from the spirit of the invention.

### Description of the Figures

- Figure 1: Fig. 1 shows a Western Blot analyses of 20 lung cancer tissue lysates. 15 µg total protein cancer (CA) tissue lysates and matched control tissue lysates were analyzed as described in example 3. M = molecular weight marker; T = tumor tissue lysate; N = matched control tissue lysate; rec ag = recombinantly produced flap endonuclease-1 (= FEN1); arrows indicate the position of FEN1.
- Figure 2: Fig. 2 shows the plot of the receiver operator characteristics (ROC-plot) of FEN1 in human lung cancer (LC) samples with an AUC of 0.87 for the assessment of 365 samples obtained from patients with LC as compared to 50 control samples obtained from obviously healthy individuals.
- Figure 3: Fig. 3 shows the plot of the receiver operator characteristics (ROC-plot) of FEN1 in human head and neck cancer (H/NC) samples with an AUC of 0.92 for the assessment of 30 samples obtained from patients with H/NC as compared to 50 control samples obtained from obviously healthy individuals.
- Figure 4: Fig. 4 shows the plot of the receiver operator characteristics (ROC-plot) of FEN1 in human endometrial cancer (EC) samples with an AUC of 0.92 for the assessment of 23 samples obtained from patients with EC as compared to 50 control samples obtained from obviously healthy individuals.
- Figure 5: Fig. 5 shows the plot of the receiver operator characteristics (ROC-plot) of FEN1 in human ovarian cancer (OC) samples with an AUC of 0.79 for the assessment of 41 samples obtained from patients with OCas compared to 50 control samples obtained from obviously healthy individuals.
- Figure 6: Fig. 6 shows the plot of the receiver operator characteristics (ROC-plot) of FEN1 in human malignant melanoma (MM) samples with an AUC of 0.95 for the assessment of 16 samples obtained from patients with MM as compared to 50 control samples obtained from obviously healthy individuals.
- Figure 7: Fig. 7 shows the plot of the receiver operator characteristics (ROC-plot) of FEN1 in human breast cancer (BC) samples with an AUC of 0.79 for the assessment of 47 samples obtained from patients with BC as compared to 50 control samples obtained from obviously healthy individuals.
- Figure 8: Fig. 8 shows the plot of the receiver operator characteristics (ROC-plot) of FEN1 in human cervix cancer (CC) samples with an AUC of 0.88 for the assessment of 20 samples obtained from patients with CC as compared to 50 control samples obtained from obviously healthy individuals.
- Figure 9: Fig. 9 shows the plot of the receiver operator characteristics (ROC-plot) of FEN1 in human pancreas cancer (PAC) samples with an AUC of 0.84 for the assessment of 50 samples obtained from patients with PAC as compared to 50 control samples obtained from obviously healthy individuals.
- Figure 10: Fig. 10 shows the plot of the receiver operator characteristics (ROC-plot) of FEN1 in human colorectal cancer (CRC) samples with an AUC of 0.79 for the assessment of 50 samples obtained from patients with CRC as compared to 50 control samples obtained from obviously healthy individuals.
- Figure 11: Fig. 11 shows the plot of the receiver operator characteristics (ROC-plot) of FEN1 in human bladder cancer (BLC) samples with an AUC of 0.76 for the assessment of 50 samples obtained from patients with BLC as compared to 50 control samples obtained from obviously healthy individuals.
- Figure 12: Fig. 12 shows the plot of the receiver operator characteristics (ROC-plot) of FEN1 in human kidney cancer (KC) samples with an AUC of 0.65 for the assessment of 25 samples obtained from patients with KC as compared to 50 control samples obtained from obviously healthy individuals.
- Figure 13: Fig. 13 shows the plot of the receiver operator characteristics (ROC-plot) of FEN1 in human prostate cancer (PC) samples with an AUC of 0.73 for the assessment of 50 samples obtained from patients with PC as compared to 50 control samples obtained from obviously healthy individuals.
- Figure 14: Fig. 14 shows the amino acid sequence of human FEN1 protein; SwissProt database accession number: P39748 (SEQ ID NO:1).

### Description of the Sequences

- SEQ ID NO: 1: shows the amino acid sequence of the human FEN1 protein according to Fig. 14; SwissProt database accession number: P39748.
- SEQ ID NO: 2: shows the synthesized peptide extension.
- SEQ ID NO: 3: shows the synthesized forward primer.
- SEQ ID NO: 4: shows the synthesized reverse primer.

### Example 1

### Identification of FEN1 as a potential marker for lung cancer

### Sources of tissue:

In order to identify tumor-specific proteins as potential diagnostic markers for lung cancer, analysis of two different kinds of tissue using proteomics methods is performed.

In total, tissue specimens from 20 patients suffering from lung cancer (LC) are analyzed. From each patient two different tissue types are collected from therapeutic resections: tumor tissue (>80% tumor) (T) and adjacent healthy tissue (N). The latter one serves as matched healthy control sample. Tissues are immediately snap frozen after resection and stored at -80°C before processing. Tumors are diagnosed by histopathological criteria.

### Tissue preparation:

0.8-1.2 g of frozen tissue are cut into small pieces, transferred to the chilled grinding jar of a mixer ball mill and completely frozen by liquid nitrogen. The tissue is pulverized in the ball mill, dissolved in the 10-fold volume (w/v) of lysis buffer (40 mM Na-citrate, 5 mM MgCl₂, 1% Genapol X-080, 0.02% Na-azide, Complete^{®} EDTA-free [Roche Diagnostics GmbH, Mannheim, Germany, Cat. No. 1 873 580]) and subsequently homogenized in a Wheaton® glass homogenizer (20 x loose fitting, 20 x tight fitting). The homogenate is subjected to centrifugation (10' at 5,000 x g), the supernatant is transferred to another vial and again subjected to centrifugation (15' at 20,000 x g). The resulting supernatant contains the soluble proteins and is used for further analysis.

### Isoelectric focussing (IEF) and SDS-PAGE:

For IEF, 3 ml of the suspension were mixed with 12 ml sample buffer (7 M urea, 2 M thiourea, 2% CHAPS, 0.4% IPG buffer pH 4-7, 0.5% DTT) and incubated for 1 h. The samples were concentrated in an Amicon^{®} Ultra-15 device (Millipore GmbH, Schwalbach, Germany) and the protein concentration was determined using the Bio-Rad^{®} protein assay (Cat.No. 500-0006; Bio-Rad Laboratories GmbH, München, Germany) following the instructions of the supplier's manual. To a volume corresponding to 1.5 mg of protein sample buffer was added to a final volume of 350 µl. This solution was used to rehydrate IPG strips pH 4-7 (Amersham Biosciences, Freiburg, Germany) overnight. The IEF was performed using the following gradient protocol: 1.) 1 minute to 500 V; 2.) 2 h to 3500 V; 3.) 22 h at constant 3500V giving rise to 82 kVh. After IEF, strips were stored at -80 °C or directly used for SDS-PAGE.

Prior to SDS-PAGE the strips were incubated in equilibration buffer (6 M urea, 50 mM Tris/HCl, pH 8.8, 30% glycerol, 2 % SDS), for reduction DDT (15 min, + 50 mg DTT/10 ml), and for alkylation IAA (15 min, + 235 mg iodacetamide/10 ml) was added. The strips were put on 12.5% polyacrylamide gels and subjected to electrophoresis at 1 W/gel for 1 h and thereafter at 17 W/gel. Subsequently, the gels were fixed (50% methanol, 10% acetate) and stained overnight with Novex^{™} Colloidal Blue Staining Kit (Invitrogen, Karlsruhe, Germany, Cat No. LC6025, 45-7101)

### Detection of FEN1 as a potential marker for human lung cancer:

Each patient was analyzed separately by image analysis with the ProteomeWeaver® software (Definiens AG, Germany, München). In addition, all spots of the gel were excised by a picking robot and the proteins present in the spots were identified by MALDI-TOF mass spectrometry (Ultraflex^{™} Tof/Tof, Bruker Daltonik GmbH, Bremen, Germany). For each patient, 3 gels from the tumor sample were compared with 3 gels each from adjacent normal tissue and analyzed for distinctive spots corresponding to differentially expressed proteins. FEN1 was identified in tumor samples of 10 patients and only in 1 control sample. By this means, protein FEN1 was found to be specifically expressed or strongly overexpressed in tumor tissue, respectively. It therefore qualified as a candidate marker for use in the diagnosis of lung cancer. The following tryptic peptides derived from FEN1 were identified:

**Table 2: Tryptic peptides identified by MALDI-TOF**

| **peptide identified** | **stretch of amino acids from FEN1 (cf. SEQ ID NO:1)** |
|---|---|
| LIADVAPSAIR | 9-19 |
| KVAIDASMSIYQFLIAVR | 30-47 |
| VAIDASMSIYQFLIAVR | 31-47 |
| QGGDVLQNEEGETTSHLMGMFYR | 48-70 |
| QLQQAQAAGAEQEVEK | 110-125 |
| KLPIQEFHLSR | 201-211 |
| LPIQEFHLSR | 202-211 |
| RAVDLIQK | 245-252 |
| LDPNKYPVPENWLHK | 263-277 |
| YPVPENWLHK | 268-277 |
| EAHQLFLEPEVLDPESVELK | 278-297 |
| WSEPNEEELIK | 298-308 |
| QFSEERIR | 315-322 |

### Example 2

### Generation of antibodies against the cancer marker protein FEN1

Polyclonal antibody to the lung cancer marker protein FEN1 is generated for further use of the antibody in the measurement of serum and plasma levels or concentrations in other body fluids of FEN1 by immunodetection assays, e. g. Western Blotting and ELISA.

### Recombinant protein expression in E. coli:

In order to generate antibodies against FEN1, the recombinant antigen is produced in *E. coli:* Therefore, the FEN1-encoding region is PCR amplified from a full-length cDNA clone obtained from the German Resource Center for Genome Research (RZPD, Berlin, Germany) using the following primers:
Forward primer (SEQ ID NO 3:) (HunI-site is underlined, coding nucleotides in capital letters).
Reverse primer (SEQ ID NO 4):
   5'-acgtacgtaagcttTCATTATTTTCCCCTTTTAAACTTC-3' (HindIII-site is underlined, coding nucleotides in capital letters).

The forward primer (besides the *Mun*I cloning and ribosomal binding sites) is encoding an N-terminal MRGSHHHHHHIEGR peptide extension (shown in SEQ ID NO: 2) fused *in-frame* at the 5'-end to the FEN1 gene. The *Mun*I/*Hind*III digested PCR fragment is ligated into the pQE80L vector (Qiagen, Hilden, Germany). Subsequently, *E.coli* XL1-blue competent cells are transformed with the generated plasmid. After sequence analysis, *E.coli* C600 competent cells are transformed with the generated plasmid for IPTG-inducible expression under control of the T5-promoter of the pQE vector series following the manufacturer's instructions.

For purification of the MRGSHHHHHHIEGR-FEN1 fusion protein, 1 L of an induced over-night bacterial culture is pelleted by centrifugation and the cell pellet is resuspended in lysis buffer (20 mM sodium-phosphate buffer, pH 7.4, 500 mM sodiumchloride (NaCl)). Cells are disupted in a French press with a pressure of 1500 bar. Insoluble material is pelleted by centrifugation (25000 g, 15 min, 4 °C) and the supernatant is applied to Ni-nitrilotriacetic acid (Ni-NTA) metal-affinity chromatography: The column is washed with several bed volumes of washing buffer (20 mM sodium-phosphate buffer, pH 7.4, 500 mM NaCl, 20 mM imidazole). Finally, bound antigen is eluted using the washing buffer with a linear gradient of 20 mM - 500 mM imidazole, antigene-containing fractions (7 mL each) are identified at O.D.₂₈₀ in an UV-detector. Antigene-containing fractions are pooled, dialyzed against storage buffer (75 mM HEPES, pH 7.5, 100 mM NaCl, 1 mM EDTA, 6.5 % (w/v) saccharose) and stored at 4 °C or -80 °C, respectively.

### Generation of peptide immunogenes for immunization:

To create polyclonal antibodies that are specific for FEN1, peptide sequences are identified that show no significant homology to other known human proteins. The amino acid sequence of FEN1 is run against the data bank of human proteins accessible at the Swiss Institute of Bioinformatics using the software Blast. The amino acid sequence 260 - 273 shows no significant homology to other human proteins and is therefore selected to raise FEN1 specific antibodies. The respective sequence is synthesized and chemically conjugated to KLH (= keyhole limpet hemocyanin) to obtain an immunogene for immunization.

### Generation of polyclonal antibodies:

### a) Immunization

For immunization, a fresh emulsion of a protein solution (100 µg/ml protein FEN1 or 500 µg/ml of KLH coupled with a peptide from the FEN1 amino acids 260 - 273) and complete Freund's adjuvant at the ratio of 1:1 is prepared. Each rabbit is immunized with 1 ml of the emulsion at days 1,7, 14 and 30, 60 and 90. Blood is drawn and resulting anti-FEN1 serum is used for further experiments as described in examples 3 and 4.

### b) Purification of IgG (immunoglobulin G) from rabbit serum by sequential precipitation with caprylic acid and ammonium sulfate

One volume of rabbit serum is diluted with 4 volumes of acetate buffer (60 mM, pH 4.0). The pH is adjusted to 4.5 with 2 M Tris-base. Caprylic acid (25 µl/ml of diluted sample) is added drop-wise under vigorous stirring. After 30 min the sample is centrifuged (13 000 x g, 30 min, 4°C), the pellet discarded and the supernatant collected. The pH of the supernatant is adjusted to 7.5 by the addition of 2 M Tris-base and filtered (0.2 µm).

The immunoglobulin in the supernatant is precipitated under vigorous stirring by the drop-wise addition of a 4 M ammonium sulfate solution to a final concentration of 2 M. The precipitated immunoglobulins are collected by centrifugation (8000 x g, 15 min, 4°C).

The supernatant is discarded. The pellet is dissolved in 10 mM NaH₂PO₄/NaOH, pH 7.5, 30 mM NaCl and exhaustively dialyzed. The dialysate is centrifuged (13 000 x g, 15 min, 4°C) and filtered (0.2 µm).

### Biotinylation of polyclonal rabbit IgG:

Polyclonal rabbit IgG is brought to 10 mg/ml in 10 mM NaH₂PO₄/NaOH, pH 7.5, 30 mM NaCl. Per ml IgG solution 50 µl Biotin -N-hydroxysuccinimide (3.6 mg/ml in DMSO) are added. After 30 min at room temperature, the sample is chromatographed on Superdex 200 (10 mM NaH₂PO₄/Na pH 7.5, 30 mM NaCl). The fraction containing biotinylated IgG are collected. Monoclonal antibodies have been biotinylated according to the same procedure.

### Digoxygenylation of polyclonal rabbit IgG:

Polyclonal rabbit IgG is brought to 10 mg/ml in 10 mM NaH₂PO₄/NaOH, 30 mM NaCl, pH 7.5. Per ml IgG solution 50 µl digoxigenin-3-O-methylcarbonyl-ε-aminocaproic acid-N-hydroxysuccinimide ester (Roche Diagnostics, Mannheim, Germany, Cat. No. 1 333 054) (3.8 mg/ml in DMSO) are added. After 30 min at room temperature, the sample is chromatographed on Superdex® 200 (10 mM NaH₂PO₄/NaOH, pH 7.5, 30 mM NaCl). The fractions containing digoxigenylated IgG are collected. Monoclonal antibodies have been labeled with digoxigenin according to the same procedure.

### Example 3

### Western Blotting for the detection of FEN1 in human lung cancer (LC) tissue using polyclonal antibody as generated in Example 2

Tissue lysates from tumor samples and healthy control samples are prepared as described in Example 1, "Tissue preparation".

SDS-PAGE and Western-Blotting are carried out using reagents and equipment of Invitrogen, Karlsruhe, Germany. For each tissue sample tested, 15 µg of tissue lysate are diluted in reducing NuPAGE® (Invitrogen) SDS sample buffer and heated for 10 min at 95°C. Samples are run on 4-12% NuPAGE® gels (Tris-Glycine) in the MES running buffer system. The gel-separated protein mixture is blotted onto nitrocellulose membranes using the Invitrogen XCell II^{™} Blot Module (Invitrogen) and the NuPAGE^{®} transfer buffer system. The membranes are washed 3 times in PBS/0.05% Tween-20 and blocked with Roti®-Block blocking buffer (A151.1; Carl Roth GmbH, Karlsruhe, Germany) for 2 h. The primary antibody, polyclonal rabbit anti-FEN1 serum (generation described in Example 2), is diluted 1:10,000 in Roti®-Block blocking buffer and incubated with the membrane for 1 h. The membranes are washed 6 times in PBS/0.05% Tween-20. The specifically bound primary rabbit antibody is labeled with an POD-conjugated polyclonal sheep anti-rabbit IgG antibody, diluted to 10 mU/ml in 0.5 x Roti®-Block blocking buffer. After incubation for 1 h, the membranes are washed 6 times in PBS/0.05% Tween-20. For detection of the bound POD-conjugated anti-rabbit antibody, the membrane is incubated with the Lumi-Light^{PLUS} Western Blotting Substrate (Order-No. 2015196, Roche Diagnostics GmbH, Mannheim, Germany) and exposed to an autoradiographic film.

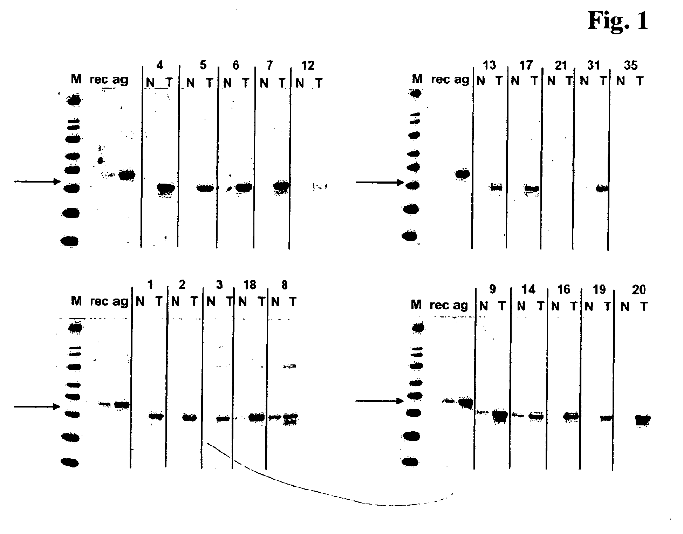

Signal intensity for FEN1 is increased in 19 out of 20 tumor tissue lysates as obtained from 20 different LC patients (Fig. 1). Thus, the increased abundance of FEN 1 in tumor tissue as detected by MALDI in example 1 is clearly confirmed by Western Blotting analyses.

### Example 4

### ELISA for the measurement of FEN1 in human serum and plasma samples or other body fluids

For detection of FEN1 in human serum or plasma, a sandwich ELISA is developed using the antibodies from example 2. For capture of the antigen the antibody against peptide 398 - 413 is conjugated with biotin while the antibodies against the FEN1 full length sequence is conjugated with digoxygenin.

For calibration of the assay HT-29 cells, a human colon carcinoma cell line included in the NCI60 tumor cell lines of the US national cancer institute, are propagated and a lysat of the cells is used for calibration. A lysat with 10.0 mg/ml is diluted to 40 µg/ml and set arbitrarily to 100 U/ml.

50 µl of a serial dilution of the HT-29 lysate as standard antigen or serum / plasma / ELF samples from patients are incubated over night with 200 µl of an antibody mix in 10 mM phosphate, pH 7.4, 1% BSA, 0,9% NaCl and 0.1% Tween 20 containing 1,25 µg/ml biotinylated anti-FEN1, aa 260 - 273, and 2.5 µg/ml digoxigenylated anti FEN-1 antibodies, respectively. Subsequently 100 µl aliquots are transferred to streptavidin-coated 96-well microtiter plates and incubated for one hour at ambient temperature. After incubation, plates are washed three times with 0.9% NaCl, 0.1% Tween 20. In a next step, wells are incubated with 100 mU/ml anti-digoxigenin-POD conjugates (Roche Diagnostics GmbH, Mannheim, Germany, Catalog No. 1633716) for 60 min in 10 mM phosphate, pH 7.4, 1% BSA, 0,9% NaCl and 0.1% Tween 20. Plates are subsequently washed three times with the same buffer. For detection of antigen-antibody complexes, wells are incubated with 100 µl TMB solution (Roche Diagnostics GmbH, Mannheim, Germany, Catalog No. 11484281001) and the OD is measured after 60 min at 450 nm with an ELISA reader.

### Example 5

### FEN1 as a serum marker for human lung cancer (LC)

Samples derived from 365 well-characterized lung cancer patients (146 adeno-CA, 87 squamous cell CA, 44 small cell CA, 88 other CA of the lung) with the UICC classification given in table 3 are used.

**Table 3: Study population**

| **Stage according to UICC** | **Number of samples** |
|---|---|
| UICC I/II | 182 |
| UICC III | 118 |
| UICC IV | 62 |
| staging unknown | 3 |
| obviously healthy blood donors | 50 |

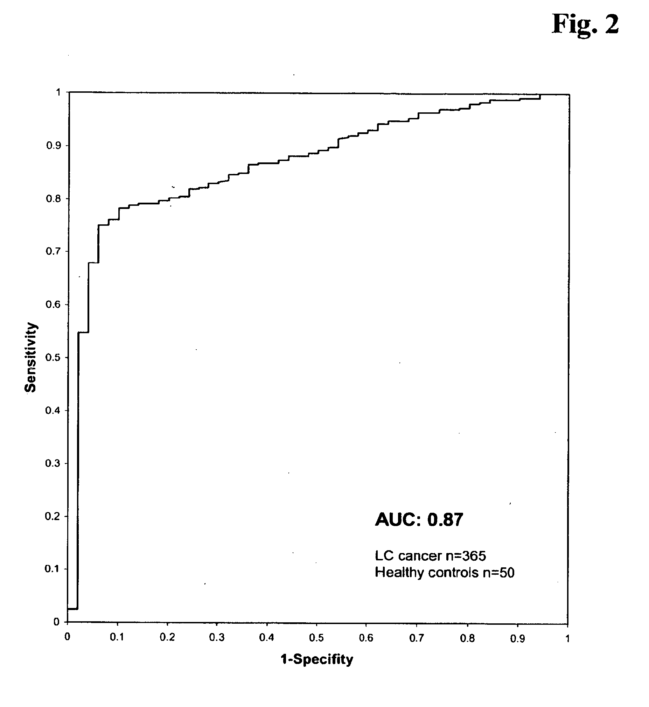

The level of FEN1 in the LC samples of Table 3 is evaluated in comparison to 50 control samples obtained from obviously healthy individuals (= control cohort), with an AUC of 0.87 (Fig. 2).

### Example 6

### FEN1 as a serum marker for human head / neck cancer (H/NC)

Samples derived from 30 well-characterized head / neck cancer patients with the UICC classification given in Table 4 are used.

**Table 4: Study population**

| **Stage according to UICC** | **Number of samples** |
|---|---|
| UICC I/II | 4 |
| UICC III | 3 |
| UICC IV | 21 |
| staging unknown | 2 |
| obviously healthy blood donors | 50 |

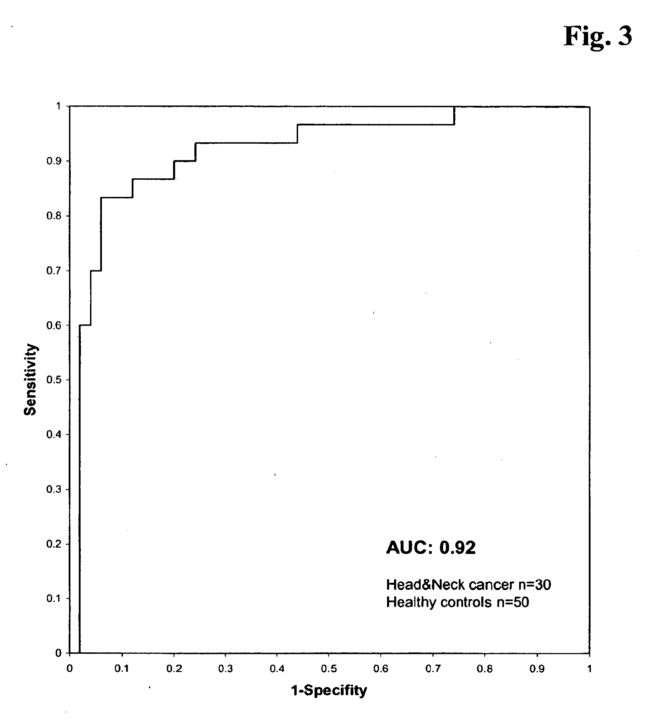

The level of FEN1 in the H/NC samples of Table 6 is evaluated in comparison to 50 control samples obtained from obviously healthy individuals (= control cohort), resulting in an AUC of 0.92 (Fig. 3).

### Example 7

### FEN1 as a serum marker for human endometrial cancer (EC)

Samples derived from 23 well-characterized endometrial cancer patients with the UICC classification given in Table 5 are used.

**Table 5: Study population**

| **Stage according to UICC** | **Number of samples** |
|---|---|
| UICC I/II | 12 |
| UICC III | 3 |
| UICC IV | 3 |
| staging unknown | 5 |
| obviously healthy blood donors | 50 |

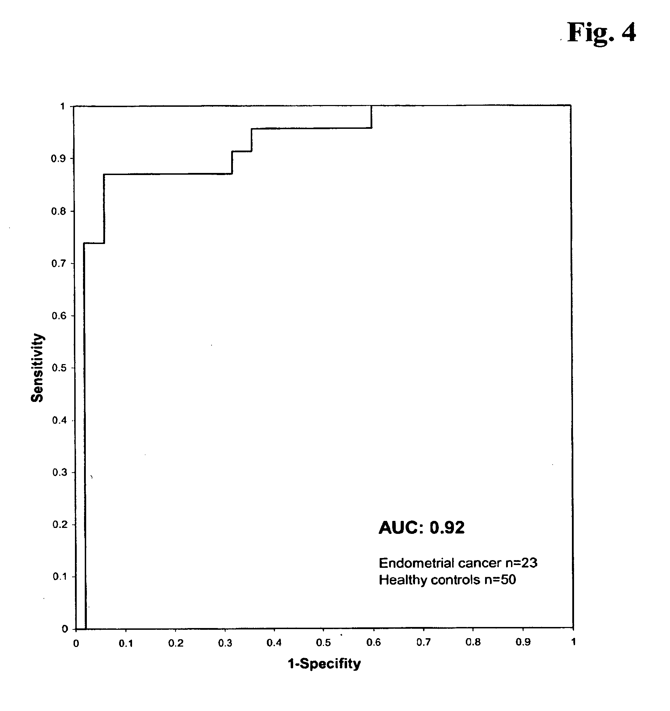

The level of FEN1 in the EC samples of Table 6 is evaluated in comparison to 50 control samples obtained from obviously healthy individuals (= control cohort), resulting in an AUC of 0.92 (Fig. 4).

### Example 8

### FEN1 as a serum marker for human ovarian cancer (OC)

Samples derived from 42 well-characterized ovarian cancer (OC) patients with the UICC classification given in Table 6 are used.

**Table 6: Study population**

| **Stage according to UICC** | **Number of samples** |
|---|---|
| UICC I/II | 7 |
| UICC III | 14 |
| UICC IV | 8 |
| staging unknown | 12 |
| obviously healthy blood donors | 50 |

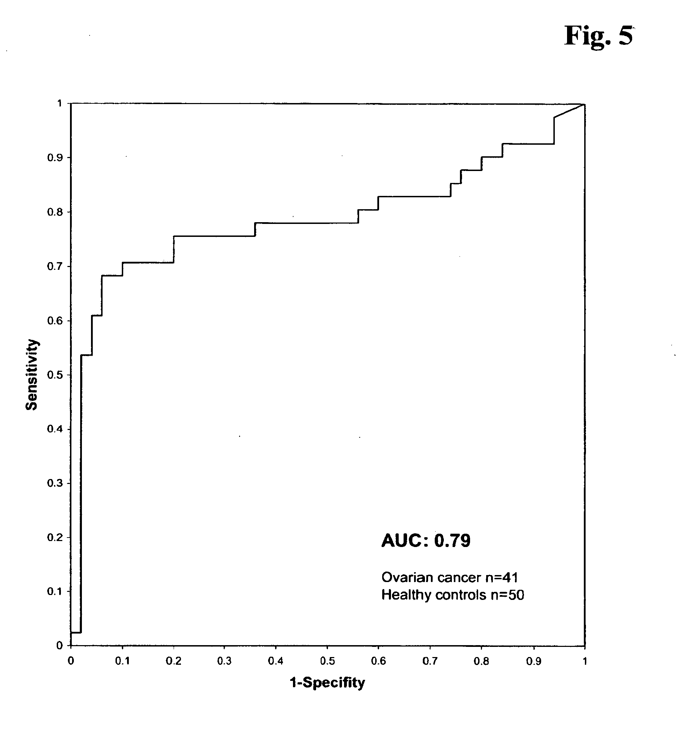

The level of FEN1 in the OC samples of Table 6 is evaluated in comparison to 50 control samples obtained from obviously healthy individuals (= control cohort), resulting in an AUC of 0.79 (Fig. 5).

### Example 9

### FEN1 as a serum marker for human malignant melanoma (MM)

Samples derived from 16 well-characterized malignant melanoma patients with the UICC classification given in Table 7 are used.

**Table 7: Study population**

| **Stage according to UICC** | **Number of samples** |
|---|---|
| UICC I/II | 3 |
| UICC III | 1 |
| UICC IV | 0 |
| staging unknown | 12 |
| obviously healthy blood donors | 50 |

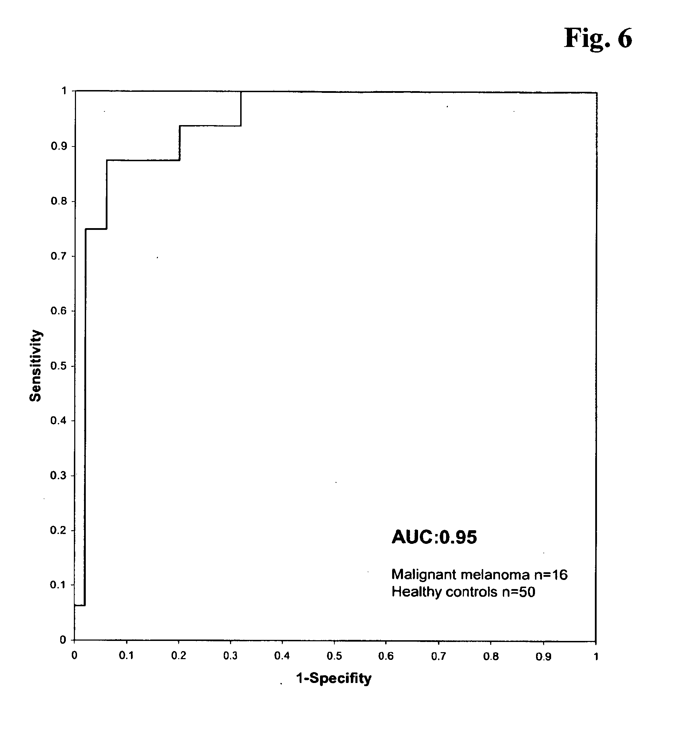

The level of FEN1 in the MM samples of Table 6 is evaluated in comparison to 50 control samples obtained from obviously healthy individuals (= control cohort), resulting in an AUC of 0.95 (Fig. 6).

### Example 10

### FEN1 as a serum marker for human breast cancer (BC)

Samples derived from 47 well-characterized breast cancer patients with the UICC classification given in Table 8 are used.

**Table 8: Study population**

| **Stage according to UICC** | **Number of samples** |
|---|---|
| UICC I/II | 26 |
| UICC III | 9 |
| UICC IV | 12 |
| obviously healthy blood donors | 50 |

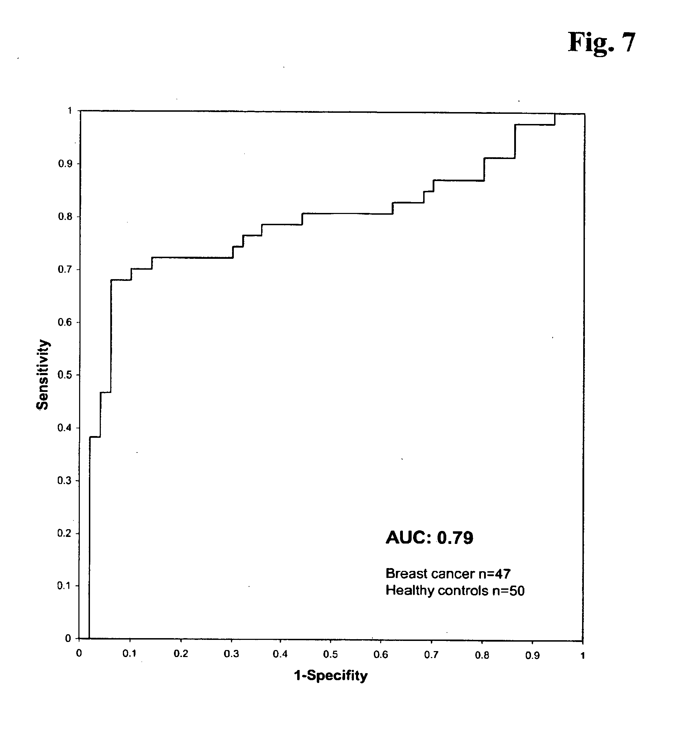

The level of FEN1 in the BC samples of Table 5 is evaluated m comparison to 50 control samples obtained from obviously healthy individuals (= control cohort), resulting in an AUC of 0.79 (Fig. 7).

### Example 11

### FEN1 as a serum marker for human cervix cancer (CC)

Samples derived from 20 well-characterized cervix cancer patients with the UICC classification given in Table 9 are used.

**Table 9: Study population**

| **Stage according to UICC** | **Number of samples** |
|---|---|
| UICC is/I/II | 11 |
| UICC III | 7 |
| UICC IV | 2 |
| staging unknown | 0 |
| obviously healthy blood donors | 50 |

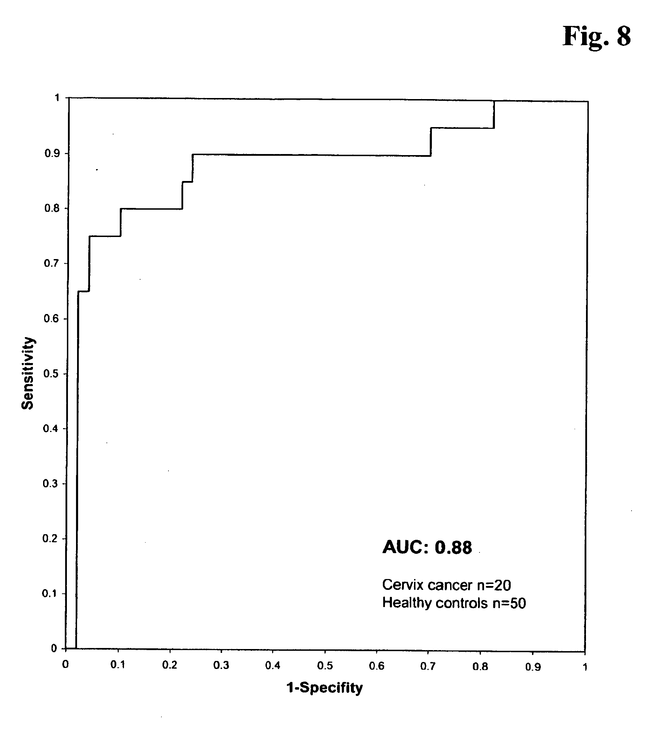

The level of FEN1 in the CC samples of Table 6 is evaluated in comparison to 50 control samples obtained from obviously healthy individuals (= control cohort), resulting in an AUC of 0.88 (Fig. 8).

### Example 12

### FEN1 as a serum marker for human pancreatic cancer (PAC)

Samples derived from 49 well-characterized prancreas cancer patients with the UICC classification given in Table 10 are used.

**Table 10: Study population**

| **Stage according to UICC** | **Number of samples** |
|---|---|
| UICC I/II | 26 |
| UICC III | 5 |
| UICC IV | 15 |
| Staging unknown | 4 |
| obviously healthy blood donors | 50 |

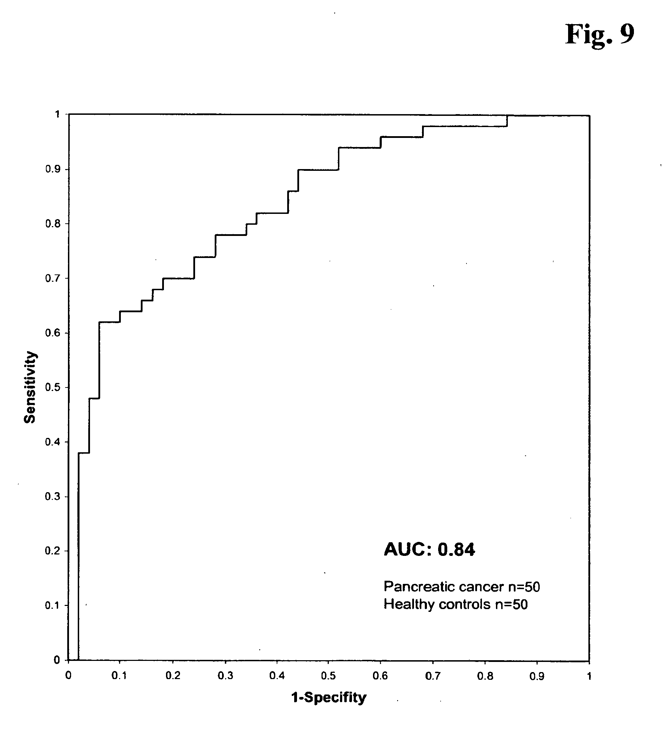

The level of FEN1 in the PAC samples of Table 6 is evaluated in comparison to 50 control samples obtained from obviously healthy individuals (= control cohort), resulting in an AUC of 0.84 (Fig. 9).

### Example 13

### FEN1 as a serum marker for human colon cancer (CRC)

Samples derived from 50 well-characterized colorectal cancer patients with the UICC classification given in Table 11 are used.

**Table 11: Study population**

| **Stage according to UICC** | **Number of samples** |
|---|---|
| UICC I/II | 25 |
| UICC III | 13 |
| UICC IV | 6 |
| staging unknown | 6 |
| obviously healthy blood donors | 50 |

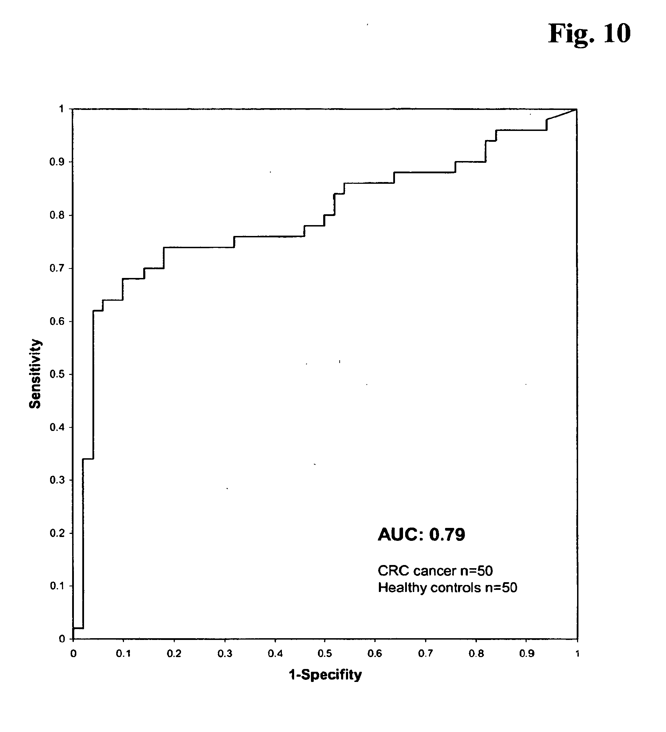

The level of FEN1 in the CRC samples of Table 4 is evaluated in comparison to 50 control samples obtained from obviously healthy individuals (= control cohort), resulting in an AUC of 0.79 (Fig. 10).

### Example 14

### FEN1 as a serum marker for human bladder cancer (BLC)

Samples derived from 50 well-characterized bladder cancer patients with the UICC classification given in Table 12 are used.

**Table 12: Study population**

| **Stage according to UICC** | **Number of samples** |
|---|---|
| UICC 0/I/II | 41 |
| UICC III | 1 |
| UICC IV | 3 |
| staging unknown | 4 |
| obviously healthy blood donors | 50 |

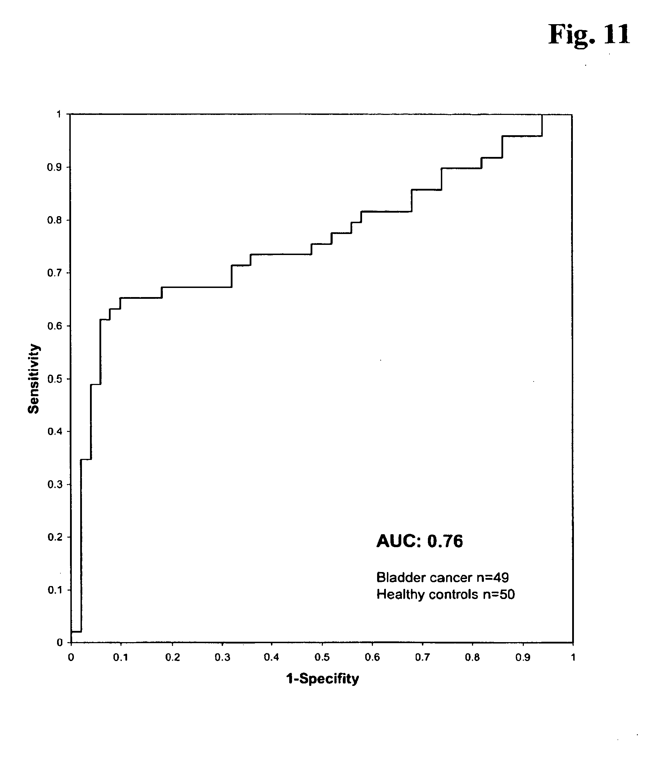

The level of FEN1 in the PC samples of Table 6 is evaluated in comparison to 50 control samples obtained from obviously healthy individuals (= control cohort), resulting in an AUC of 0.76 (Fig. 11).

### Example 15

### FEN1 as a serum marker for human kidney cancer (KC)

Samples derived from 25 well-characterized kidney cancer patients with the UICC classification given in Table 13 are used.

**Table 13: Study population**

| **Stage according to UICC** | **Number of samples** |
|---|---|
| UICC I/II | 13 |
| UICC III | 4 |
| UICC IV | 3 |
| staging unknown | 5 |
| obviously healthy blood donors | 50 |

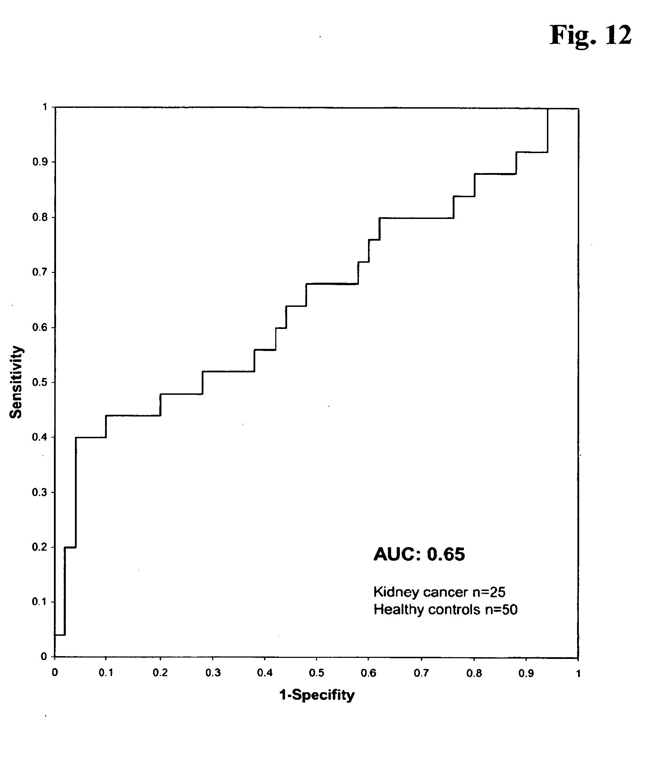

The level of FEN1 in the KC samples of Table 6 is evaluated in comparison to 50 control samples obtained from obviously healthy individuals (= control cohort), resulting in an AUC of 0.65 (Fig. 12).

### Example 16

### FEN1 as a serum marker for human prostate cancer (PC)

Samples derived from 50 well-characterized prostate cancer patients with the UICC classification given in Table 14 are used.

**Table 14: Study population**

| **Stage according to UICC** | **Number of samples** |
|---|---|
| UICC I/II | 24 |
| UICC III | 4 |
| UICC IV | 6 |
| staging unknown | 16 |
| obviously healthy blood donors | 50 |

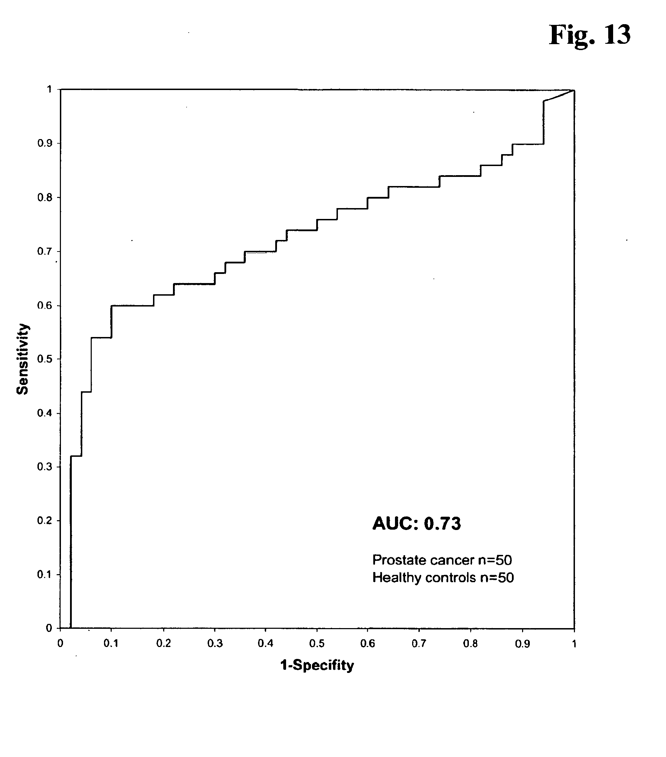

The level of FEN1 in the PC samples of Table 6 is evaluated in comparison to 50 control samples obtained from obviously healthy individuals (= control cohort), resulting in an AUC of 0.73 (Fig. 13).

### Example 17

### FEN1 in epithelial lining fluid (ELF) - bronchoscopic microsampling

Bronchoscopic microsampling (BMS) offers the possibility to collect epithelial lining fluid (ELF) near small pulmonary nodules in a largely non-invasive manner. Subsequently, it is possible to measure concentrations of tumor markers in ELF in order to identify a malignant nodule. A patient specific baseline concentration of the respective tumor marker is obtained by sampling ELF in the contralateral lung.

The BMS probe (Olympus Medical Systems Corp. Tokyo, Japan, Cat.-No.: BC-402C) is inserted into the lungs through the bronchoscope and consists of an outer polyethylene sheath and an inner cotton probe attached to a stainless steel guide. The inner probe is advanced to the proximity of the nodule and BMS is performed for a few seconds. Afterwards, the inner probe is withdrawn into the outer sheath and both devices are withdrawn simultaneously. The cotton tip is cut off and directly frozen at -80°C. For the determination, ELF is eluted from the cotton tip and can be analyzed subsequently. The concentration of FEN1 is determined in ELF with the ELISA as described in Example 4.

## Claims

1. A method for assessing cancer in vitro comprising measuring in a serum or plasma sample the concentration of
(a) Flap endonuclease-1 protein (= FEN1),
(b) optionally one or more other marker of cancer, and
(c) using the measurement result of step (a) and optionally of step (b) in the assessment of cancer, wherein an increased concentration of a FEN1 protein is indicative for cancer.

2. The method according to claims 1, wherein the said method is a sandwich immunoassay.

3. The method according to claims 1 to 2, further **characterized in that** the method is for assessing cancers like endometrial cancer, malignant melanoma, cervix cancer, head and neck cancer, ovarian cancer, colon cancer, bladder cancer, pancreatic cancer, breast cancer, small cell lung cancer, prostate cancer, kidney cancer and non small cell lung cancer.

4. The method according to any one of claims 1 to 3, further **characterized in that** said one or more other marker of step (b) is selected from the group consisting of CEA, NSE, CA 19-9, CA 125, PSA, proGRP, SCC, NNMT, anti-p53 autoantibodies, Seprase and DPPIV/Seprase.

5. The method according any one of claims 1 to 4, further **characterized in that** the concentration is measured by an immunological method.

6. Use of FEN1 protein in the in vitro assessment of cancer, wherein an increased concentration of a FEN1 protein is indicative for cancer, and wherein FEN1 protein is detected in a serum or plasma sample.

7. The use according to claim 6 in the assessment of a cancer selected from the group consisting of endometrial cancer, malignant melanoma, cervix cancer, head and neck cancer, ovarian cancer, colon cancer, bladder cancer, pancreatic cancer, breast cancer, small cell lung cancer, prostate cancer, kidney cancer and non small cell lung cancer.

8. The use of an antibody directed against FEN1 protein in the in vitro assessment of cancer, wherein an increased concentration of FEN1 protein in a serum or plasma sample is indicative for cancer.

9. The use of a marker panel comprising FEN1 protein and optionally one or more other marker for cancer in the in vitro assessment of cancer, wherein an increased concentration of FEN1 protein in a serum or plasma sample is indicative for cancer.

10. The use of the marker panel according to claim 9, further **characterized in that** the optionally one or more other marker is selected from the group consisting of CEA, NSE, CA 19-9, CA 125, PSA, proGRP, SCC, NNMT, anti-p53 autoantibodies, Seprase and DPPIV/Seprase.

11. The use of the marker panel according to any of the claims 9 and 10 in the assessment of endometrial cancer, malignant melanoma, cervix cancer, head and neck cancer, ovarian cancer, colon cancer, bladder cancer, pancreatic cancer, breast cancer, small cell lung cancer, prostate cancer, kidney cancer and non small cell lung cancer.

## Patentansprüche

1. Verfahren zur Bewertung von Krebs in vitro, bei dem man in einer Serum- oder Plasmaprobe die Konzentration von
(a) Flap-Endonuklease-1-Protein (=FEN1),
(b) gegebenenfalls einem anderen Krebsmarker oder mehreren anderen Krebsmarkern misst und
(c) das Messergebnis von Schritt (a) und gegebenenfalls von Schritt (b) zur Bewertung von Krebs verwendet, wobei eine erhöhte Konzentration eines FEN1-Proteins indikativ für Krebs ist.

2. Verfahren nach Anspruch 1, wobei es sich bei dem Verfahren um ein Sandwich-Immunassay handelt.

3. Verfahren nach Anspruch 1 oder 2, weiterhin **dadurch gekennzeichnet, dass** das Verfahren zur Bewertung von Krebstypen wie Endometriumkarzinom, malignem Melanom, Gebärmutterhalskrebs, Kopf- und Halskrebs, Eierstockkrebs, Dickdarmkrebs, Blasenkrebs, Bauchspeicheldrüsenkrebs, Brustkrebs, kleinzelligem Lungenkrebs, Prostatakrebs, Nierenkrebs und nichtkleinzelligem Lungenkrebs bestimmt ist.

4. Verfahren nach einem der Ansprüche 1 bis 3, weiterhin **dadurch gekennzeichnet, dass** der eine oder die mehreren anderen Marker in Schritt (b) ausgewählt sind aus der Gruppe bestehend aus CEA, NSE, CA 19-9, CA 125, PSA, proGRP, SCC, NNMT, Anti-p53-Autoantikörpern, Seprase und DPPIV/Seprase.

5. Verfahren nach einem der Ansprüche 1 bis 4, weiterhin **dadurch gekennzeichnet, dass** die Konzentration durch ein immunologisches Verfahren gemessen wird.

6. Verwendung von FEN1-Protein bei der in-vitro-Bewertung von Krebs, wobei eine erhöhte Konzentration eines FEN1-Proteins indikativ für Krebs ist, und wobei FEN1-Protein in einer Serum- oder Plasmaprobe nachgewiesen wird.

7. Verwendung nach Anspruch 6 zur Bewertung eines Krebstyps ausgewählt aus der Gruppe bestehend aus Endometriumkarzinom, malignem Melanom, Gebärmutterhalskrebs, Kopf- und Halskrebs, Eierstockkrebs, Dickdarmkrebs, Blasenkrebs, Bauchspeicheldrüsenkrebs, Brustkrebs, kleinzelligem Lungenkrebs, Prostatakrebs, Nierenkrebs und nichtkleinzelligem Lungenkrebs.

8. Verwendung eines gegen FEN1-Protein gerichteten Antikörpers bei der in-vitro-Bewertung von Krebs, wobei eine erhöhte Konzentration an FEN1-Protein in einer Serum- oder Plasmaprobe indikativ für Krebs ist.

9. Verwendung eines FEN1-Protein und gegebenenfalls einen oder mehrere andere Marker für Krebs umfassenden Markerpanels bei der in-vitro-Bewertung von Krebs, wobei eine erhöhte Konzentration an FEN1-Protein in einer Serum- oder Plasmaprobe indikativ für Krebs ist.

10. Verwendung des Markerpanels nach Anspruch 9, weiterhin **dadurch gekennzeichnet, dass** der eine oder die mehreren anderen gegebenenfalls vorhandenen Marker ausgewählt sind aus der Gruppe bestehend aus CEA, NSE, CA 19-9, CA 125, PSA, proGRP, SCC, NNMT, Anti-p53-Autoantikörpern, Seprase und DPPIV/Seprase.

11. Verwendung des Markerpanels nach Anspruch 9 oder 10 zur Bewertung von Endometriumkarzinom, malignem Melanom, Gebärmutterhalskrebs, Kopf- und Halskrebs, Eierstockkrebs, Dickdarmkrebs, Blasenkrebs, Bauchspeicheldrüsenkrebs, Brustkrebs, kleinzelligem Lungenkrebs, Prostatakrebs, Nierenkrebs und nichtkleinzelligem Lungenkrebs.

## Revendications

1. Procédé d'évaluation du cancer *in vitro* comprenant la mesure dans un prélèvement de sérum ou de plasma de la concentration de
(a) la protéine endonucléase Flap-1 (= FEN1),
(b) éventuellement un ou plusieurs autre(s) marqueur(s) du cancer, et
(c) l'utilisation du résultat de la mesure de l'étape (a) et éventuellement de l'étape (b) dans l'évaluation du cancer, dans laquelle une concentration accrue d'une protéine FEN1 est indicatrice du cancer.

2. Procédé selon la revendication 1, dans lequel ledit procédé est un dosage immunologique de type sandwich.

3. Procédé selon les revendications 1 à 2, **caractérisé en outre en ce que** le procédé sert à l'évaluation de cancers tels que le cancer de l'endomètre, le mélanome malin, le cancer du col, le cancer de la tête et du cou, le cancer de l'ovaire, le cancer du côlon, le cancer de la vessie, le cancer du pancréas, le cancer du sein, le cancer du poumon à petites cellules, le cancer de la prostate, le cancer rénal et le cancer du poumon non à petites cellules.

4. Procédé selon l'une quelconque des revendications 1 à 3, **caractérisé en outre en ce que** ledit(lesdits) un ou plusieurs autre(s) marqueur(s) de l'étape (b) est(sont) sélectionné(s) parmi le groupe constitué de CEA, NSE, CA 19-9, CA 125, PSA, proGRP, SCC, NNMT, des auto-anticorps anti-p53, de la séprase et de la DPPIV/séprase.

5. Procédé selon l'une quelconque des revendications 1 à 4, **caractérisé en outre en ce que** la concentration est mesurée grâce à un procédé immunologique.

6. Utilisation de la protéine FEN1 dans l'évaluation *in vitro* du cancer, dans lequel une concentration accrue d'une protéine FEN1 est indicatrice du cancer, et dans laquelle la protéine FEN1 est détectée dans un prélèvement de sérum ou de plasma.

7. Utilisation selon la revendication 6 dans l'évaluation d'un cancer sélectionné parmi le groupe constitué du cancer de l'endomètre, du mélanome malin, du cancer du col, du cancer de la tête et du cou, du cancer de l'ovaire, du cancer du côlon, du cancer de la vessie, du cancer du pancréas, du cancer du sein, du cancer du poumon à petites cellules, du cancer de la prostate, du cancer rénal et du cancer du poumon non à petites cellules.

8. Utilisation d'un anticorps dirigé contre la protéine FEN1 dans l'évaluation *in vitro* du cancer, dans lequel une concentration accrue de la protéine FEN1 dans un prélèvement de sérum ou de plasma est indicatrice du cancer.

9. Utilisation d'un panel de marqueurs comprenant la protéine FEN1 et éventuellement d'un ou plusieurs autre(s) marqueur(s) du cancer dans l'évaluation *in vitro* du cancer, dans laquelle une concentration accrue de la protéine FEN1 dans un prélèvement de sérum ou de plasma est indicatrice du cancer.

10. Utilisation du panel de marqueurs selon la revendication 9, **caractérisée en outre en ce que** le un ou plusieurs autre(s) marqueur(s) facultatif(s) est(sont) sélectionné(s) parmi le groupe constitué de CEA, NSE, CA 19-9, CA 125, PSA, proGRP, SCC, NNMT, des auto-anticorps anti-p53, de la séprase et de la DPPIV/séprase.

11. Utilisation du panel de marqueurs selon l'une quelconque des revendications 9 et 10 dans l'évaluation du cancer de l'endomètre, du mélanome malin, du cancer du col, du cancer de la tête et du cou, du cancer de l'ovaire, du cancer du côlon, du cancer de la vessie, du cancer du pancréas, du cancer du sein, du cancer du poumon à petites cellules, du cancer de la prostate, du cancer rénal et du cancer du poumon non à petites cellules.


### SEQUENCE LISTING

<110> Roche Diagnostics GmbH
   F. Hoffmann-La Roche AG
<120> Flap endonuclease-1 as a marker for cancer
<130> 26208 WO
<150> EP09165636.3
   <151> 2009-07-16
<160> 4
<170> PatentIn version 3.3
<210> 1
   <211> 380
   <212> PRT
   <213> Homo sapiens
<400> 1
   Met Gly Ile Gln Gly Leu Ala Lys Leu Ile Ala Asp Val Ala Pro Ser
   1               5                   10                  15
   Ala Ile Arg Glu Asn Asp Ile Lys Ser Tyr Phe Gly Arg Lys Val Ala
               20                  25                  30
   Ile Asp Ala Ser Met Ser Ile Tyr Gln Phe Leu Ile Ala Val Arg Gln
           35                  40                  45
   Gly Gly Asp Val Leu Gln Asn Glu Glu Gly Glu Thr Thr Ser His Leu
       50                  55                  60
   Met Gly Met Phe Tyr Arg Thr Ile Arg Met Met Glu Asn Gly Ile Lys
   65                  70                  75                  80
   Pro Val Tyr Val Phe Asp Gly Lys Pro Pro Gln Leu Lys Ser Gly Glu
                   85                  90                  95
   Leu Ala Lys Arg Ser Glu Arg Arg Ala Glu Ala Glu Lys Gln Leu Gln
               100                 105                 110
   Gln Ala Gln Ala Ala Gly Ala Glu Gln Glu Val Glu Lys Phe Thr Lys
           115                 120                 125
   Arg Leu Val Lys Val Thr Lys Gln His Asn Asp Glu Cys Lys His Leu
       130                 135                 140
   Leu Ser Leu Met Gly Ile Pro Tyr Leu Asp Ala Pro Ser Glu Ala Glu
   145                 150                 155                 160
   Ala Ser Cys Ala Ala Leu Val Lys Ala Gly Lys Val Tyr Ala Ala Ala
                   165                 170                 175
   Thr Glu Asp Met Asp Cys Leu Thr Phe Gly Ser Pro Val Leu Met Arg
               180                 185                 190
   His Leu Thr Ala Ser Glu Ala Lys Lys Leu Pro Ile Gln Glu Phe His
           195                 200                 205
   Leu Ser Arg Ile Leu Gln Glu Leu Gly Leu Asn Gln Glu Gln Phe Val
       210                 215                 220
   Asp Leu Cys Ile Leu Leu Gly Ser Asp Tyr Cys Glu Ser Ile Arg Gly
   225                 230                 235                 240
   Ile Gly Pro Lys Arg Ala Val Asp Leu Ile Gln Lys His Lys Ser Ile
                   245                 250                 255
   Glu Glu Ile Val Arg Arg Leu Asp Pro Asn Lys Tyr Pro Val Pro Glu
               260                 265                 270
   Asn Trp Leu His Lys Glu Ala His Gln Leu Phe Leu Glu Pro Glu Val
           275                 280                 285
   Leu Asp Pro Glu Ser Val Glu Leu Lys Trp Ser Glu Pro Asn Glu Glu
       290                 295                 300
   Glu Leu Ile Lys Phe Met Cys Gly Glu Lys Gln Phe Ser Glu Glu Arg
   305                 310                 315                 320
   Ile Arg Ser Gly Val Lys Arg Leu Ser Lys Ser Arg Gln Gly Ser Thr
                   325                 330                 335
   Gln Gly Arg Leu Asp Asp Phe Phe Lys Val Thr Gly Ser Leu Ser Ser
               340                 345                 350
   Ala Lys Arg Lys Glu Pro Glu Pro Lys Gly Ser Thr Lys Lys Lys Ala
           355                 360                 365
   Lys Thr Gly Ala Ala Gly Lys Phe Lys Arg Gly Lys
       370                 375                 380
<210> 2
   <211> 14
   <212> PRT
   <213> Artificial
<220>
   <223> Synthesized Peptide Extension
<400> 2
   Met Arg Gly Ser His His His His His His Ile Glu Gly Arg
   1               5                   10
<210> 3
   <211> 93
   <212> DNA
   <213> Artificial
<220>
   <223> Synthesized Forward Primer
<400> 3
<210> 4
   <211> 39
   <212> DNA
   <213> Artificial
<220>
   <223> Synthesized Reverse Primer
<400> 4
   acgtacgtaa gctttcatta ttttcccctt ttaaacttc
   39